# EUROPEAN PATENT APPLICATION

(11) **EP 2 072 060 A1**
(43) Date of publication of application: **24.06.2009**
(21) Application number: 07301691.7
(22) Date of filing: 18.12.2007
(51) Int. Cl.: A61K 47/48

(54) **Methods and compositions for the preparation and use of toxin conjugates.**

(71) Applicant: Institut Curie, 75248 Paris Cedex 05 (FR); CENTRE NATIONAL DE LA RECHERCHE SCIENTIFIQUE (CNRS), 75794 Paris Cedex 16 (FR)
(72) Inventor: LUDGER, Johannes, 92400, COURBEVOIE (FR); GIOVANI, Baldissera, 75013, PARIS (FR); AZOULAY, Michel, 75006, PARIS (FR); FLORENT, Jean-Claude, 91190, GIF SUR YVETTE (FR)
(74) Representative: Cabinet Plasseraud

(57) **Abstract**

The present invention relates to methods for the preparation of toxin conjugates that are useful in vaccination and other therapies and diagnostics. In particular, methods are provided that involve a [3+2] cycloaddition between a first reactive unsaturated group on a toxin moiety and a second reactive unsaturated group on a bioactive moiety. Also provided are conjugates that are formed through this conjugation method, pharmaceutical compositions comprising these conjugates, and methods of using these pharmaceutical compositions for the treatment or diagnostic of antigen-related conditions, including tumors and infections.

SEQUENCE LISTING

<110>  INSTITUT CURIE
       CNRS
       LUDGER, Johannes

<120>  Methods and compositions for the preparation and use of toxin
       conjugates

<130>  EP07301691.7

<160>  1

<170>  PatentIn version 3.3

<210>  1
<211>  636
<212>  PRT
<213>  homo sapiens

<400>  1

Cys Thr Gln Val Cys Thr Gly Thr Asp Met Lys Leu Arg Leu Pro Ala
1               5                   10                  15

Ser Pro Glu Thr His Leu Asp Met Leu Arg His Leu Tyr Gln Gly Cys
            20                  25                  30

Gln Val Val Gln Gly Asn Leu Glu Leu Thr Tyr Leu Pro Thr Asn Ala
        35                  40                  45

Ser Leu Ser Phe Leu Gln Asp Ile Gln Glu Val Gln Gly Tyr Val Leu
    50                  55                  60

Ile Ala His Asn Gln Val Arg Gln Val Pro Leu Gln Arg Leu Arg Ile
65                  70                  75                  80

Val Arg Gly Thr Gln Leu Phe Glu Asp Asn Tyr Ala Leu Ala Val Leu
                85                  90                  95

Asp Asn Gly Asp Pro Leu Asn Asn Thr Thr Pro Val Thr Gly Ala Ser
            100                 105                 110

Pro Gly Gly Leu Arg Glu Leu Gln Leu Arg Ser Leu Thr Glu Ile Leu
        115                 120                 125

Lys Gly Gly Val Leu Ile Gln Arg Asn Pro Gln Leu Cys Tyr Gln Asp
    130                 135                 140

Thr Ile Leu Trp Lys Asp Ile Phe His Lys Asn Asn Gln Leu Ala Leu
145                 150                 155                 160

Thr Leu Ile Asp Thr Asn Arg Ser Arg Ala Cys His Pro Cys Ser Pro
                165                 170                 175

Met Cys Lys Gly Ser Arg Cys Trp Gly Glu Ser Ser Glu Asp Cys Gln
            180                 185                 190

Ser Leu Thr Arg Thr Val Cys Ala Gly Gly Cys Ala Arg Cys Lys Gly
        195                 200                 205

Pro Leu Pro Thr Asp Cys Cys His Glu Gln Cys Ala Ala Gly Cys Thr
    210                 215                 220

Gly Pro Lys His Ser Asp Cys Leu Ala Cys Leu His Phe Asn His Ser
225                 230                 235                 240

Gly Ile Cys Glu Leu His Cys Pro Ala Leu Val Thr Tyr Asn Thr Asp
                245                 250                 255

Thr Phe Glu Ser Met Pro Asn Pro Glu Gly Arg Tyr Thr Phe Gly Ala
            260                 265                 270

Ser Cys Val Thr Ala Cys Pro Tyr Asn Tyr Leu Ser Thr Asp Val Gly
        275                 280                 285

Ser Cys Thr Leu Val Cys Pro Leu His Asn Gln Glu Val Thr Ala Glu
    290                 295                 300

Asp Gly Thr Gln Arg Cys Glu Lys Cys Ser Lys Pro Cys Ala Arg Val
305                 310                 315                 320

Cys Tyr Gly Leu Gly Met Glu His Leu Arg Glu Val Arg Ala Val Thr
                325                 330                 335

Ser Ala Asn Ile Gln Glu Phe Ala Gly Cys Lys Lys Ile Phe Gly Ser
            340                 345                 350

Leu Ala Phe Leu Pro Glu Ser Phe Asp Gly Asp Pro Ala Ser Asn Thr
        355                 360                 365

Ala Pro Leu Gln Pro Glu Gln Leu Gln Val Phe Glu Thr Leu Glu Glu
    370                 375                 380

Ile Thr Gly Tyr Leu Tyr Ile Ser Ala Trp Pro Asp Ser Leu Pro Asp
385                 390                 395                 400

Leu Ser Val Phe Gln Asn Leu Gln Val Ile Arg Gly Arg Ile Leu His
                405                 410                 415

Asn Gly Ala Tyr Ser Leu Thr Leu Gln Gly Leu Gly Ile Ser Trp Leu
            420                 425                 430

Gly Leu Arg Ser Leu Arg Glu Leu Gly Ser Gly Leu Ala Leu Ile His
        435                 440                 445

His Asn Thr His Leu Cys Phe Val His Thr Val Pro Trp Asp Gln Leu
    450                 455                 460

Phe Arg Asn Pro His Gln Ala Leu Leu His Thr Ala Asn Arg Pro Glu
465                 470                 475                 480

Asp Glu Cys Val Gly Glu Gly Leu Ala Cys His Gln Leu Cys Ala Arg
                485                 490                 495

Gly His Cys Trp Gly Pro Gly Pro Thr Gln Cys Val Asn Cys Ser Gln
            500                 505                 510

Phe Leu Arg Gly Gln Glu Cys Val Glu Glu Cys Arg Val Leu Gln Gly
        515                 520                 525

Leu Pro Arg Glu Tyr Val Asn Ala Arg His Cys Leu Pro Cys His Pro
    530                 535                 540

Glu Cys Gln Pro Gln Asn Gly Ser Val Thr Cys Phe Gly Pro Glu Ala
545                 550                 555                 560

Asp Gln Cys Val Ala Cys Ala His Tyr Lys Asp Pro Pro Phe Cys Val
                565                 570                 575

Ala Arg Cys Pro Ser Gly Val Lys Pro Asp Leu Ser Tyr Met Pro Ile
            580                 585                 590

Trp Lys Phe Pro Asp Glu Glu Gly Ala Cys Gln Pro Cys Pro Ile Asn
        595                 600                 605

Cys Thr His Ser Cys Val Asp Leu Asp Asp Lys Gly Cys Pro Ala Glu
    610                 615                 620

Gln Arg Ala Ser Pro Leu His His His His His His
625                 630                 635

## Description

### Background of the Invention

A number of protein toxins of bacterial and plant origin reach the cytosol of eukaryotic target cells by complex mechanisms involving receptor binding, membrane interaction and translocation across a cell lipid membrane. The study of these toxins has provided essential information about mechanisms that can be used to gain access to the cytosol as well as detailed knowledge about endocytosis and intracellular sorting. Research efforts have focussed on exploiting the intracellular trafficking properties of toxins. Membrane interaction and ability to reach the cytoplasm have been used to present proteins at the cell surface and to transport foreign peptides or nucleotides into the cytoplasm, respectively. These approaches can find applications, for example, in anticancer vaccination and in inducing a major histocompatibility (MHC) class I presentation of exogenous peptides or proteins (R.S. Lee et al., Eur. J. Immunol., 1998, 28: 2726-2737; K.L. Noakes et al., FEBS Lett., 1999, 453: 95-99; N. Haicheur et al., J; Immunol., 2000, 165: 3301-3308). Examples of toxins (or non-toxic versions thereof) that have been investigated as vectors or drug delivery systems for the development of new strategies in vaccination and therapeutic methods include anthrax toxin, pertussin toxin, diphtheria toxin, chlorotoxin, botulinum toxin, cholera toxin, *Escherichia coli* heat-labile enterotoxin, and pH-sensitive toxins.

Another example of such toxins is the Shiga toxin, a bacterial toxin of the AB5 family that is secreted by the disentry-causing bacterium *Shigella dysenteriae* (K. Sandvig and B. van Deurs, Annu. Rev. Cell Devel. Biol., 2002, 18: 1-24; J. Gariepy, Crit. Rev. Oncol./Hematol., 2001, 39: 99-106; K. Sandvig, Toxicon., 2001, 39: 1629-1635; and D.G. Pina and L. Johannes, Toxicon., 2005, 45: 389-393). Shiga toxin is composed of an enzymatic A-subunit and a non-toxic B-subunit. The A-subunit modifies ribosomal RNA thus leading to inhibition of protein synthesis in higher eukaryotic target cells after transfer into the cytoplasm of these cells. For cellular binding and intracellular transport, the A-subunit has to interact with the B-subunit. The B-subunit is a homopentamer protein that mediates binding to and internalization into target cells by interacting with the cell surface glycophingolipid receptor, globotriaosyl ceramide, Gb3, also called CD77 (L. Johannes and B. Goud, Trends Cell Biol., 1998, 8: 158-162). The B-subunit alone is non-toxic, but conserves the intracellular transport characteristics of the holotoxin which, in many Gb3-expressing cells, is transported in a retrograde fashion from the plasma membrane to the endoplasmic reticulum, *via* the early endosome and the Golgi apparatus. At the level of the endoplasmic reticulum, the A-subunit then passes *via* retro-translocation across the membrane into the cytosol.

The retrograde trafficking pathway from endosomes to the Golgi apparatus and endoplasmic reticulum is of special importance since it provides a route to deliver therapeutic molecules, bypassing the acidic hydrolytic environment of the lysosomes. Furthermore, Gb3 is known to be specifically expressed on human and mouse dendritic cells, which are sentinels of the immune system (T. Falguières et al., Mol. Biol. Cell, 2001, 12: 2453-2468). The Gb3 receptor has also been reported to be expressed preferentially in some ectodermic derived tumors (plasma) and Burkitt's lymphomas.

The present Applicants have previously shown that a CD8 human tumor antigen fused to the B-subunit of Shiga toxin (STxB) could efficiently be presented in a human leukocyte antigen (HLA) class I-restricted manner to specific cytotoxic T lymphocytes (CTL) (R.S. Lee et al., Eur. J. Immunol., 1998, 28: 2726-2737). This result was independently confirmed by another study which demonstrated that Shiga holotoxin, carrying a defined peptide epitope from influenza virus, could deliver the antigen into the MHC class I intracellular pathway (K.L. Noakes *et al.,* FEBS Lett., 1999, 453: 95). By incubating dendritic cells with full-size antigenic proteins, or fragments thereof, the Applicants have shown that peptides derived from these antigenic proteins were presented by both MHC class I and II molecules (N. Haicheur et al., J. Immunol., 2000, 165: 3301-3308). Vaccination of mice with STxB coupled to different full-size antigenic proteins, or peptides thereof, was also demonstrated to induce a CTL response (N. Haicheur et al., J. Immunol., 2003, 15: 1161-1171; B. Vingert et al., Eur. J. Immunol., 2006, 36: 1124-1135).

The present Applicants have also shown that fusion proteins between STxB and an antigen, or an epitope from a model tumor antigen, can elicit specific cytotoxic T lymphocytes response (CLT), whereas each moiety of said fusion proteins did not lead individually to CTL induction (R.S. Lee et al., Eur. J. Immunol., 1998, 28: 2726-2737; N. Haicheur et al., J. Immunol., 2000, 165: 3301-3308; U.S. Publication No. 2004/0110935; and European Patent No. EP 1 355 928).

The main difficulty in this technology is that, for each application, i.e., for each antigen or fragment thereof, it is necessary to specifically construct a fusion protein, which requires specific construction of a recombinant vector bearing the sequences encoding this fusion protein to be expressed in a host cell. Alternatively, a toxin-antigen conjugate may be prepared chemically. Classical chemical methods for the preparation of such protein conjugates require the use of heterobifunctional crosslinking agents, such as N-succinimidyl m-(N-maleimido)benzoate (SMBS). In such methods, the antigenic protein is first modified *via* its primary amino groups, mostly on Lysine, using the succinimidyl ester functionality of the crosslinker. In a second step, the maleimide moiety of the crosslinker is reacted with free sulfhydryl groups on STxB-Cys, a STxB variant with a thiol functionality. Although this chemical approach has allowed the efficient formation of different STxB-antigen conjugates, it exhibits important limitations. In particular, since several free amino groups are present on antigenic proteins, the reaction products are generally heterogeneous, and batch to batch variability is difficult to avoid. Furthermore, Lysine residues are often present in antigenic peptides, and their modification by SMBS may render the corresponding peptides inactive.

Clearly, improved techniques are still needed for the preparation of toxin conjugates of therapeutic relevance. In particular, the development of preparation methods that are chemoselective remains highly desirable.

### Summary of the Invention

The present invention is directed to improved systems and strategies for the preparation of toxin conjugates that are useful in vaccination and other therapeutic and diagnostic applications. In particular, the present invention provides methods of conjugation for the formation of toxin conjugates which include a [3+2] cycloaddition that is selective and stereospecific, and that allows for reproducible preparation of toxin conjugates.

More specifically, in one aspect, the present invention provides toxin conjugates comprising at least one toxin moiety covalently bound to at least one bioactive moiety, wherein covalent binding between the toxin moiety and bioactive moiety results from a [3+2] cycloaddition. In such methods, the [3+2] cycloaddition generally occurs between a first reactive unsaturated group on the toxin moiety and a second reactive unsaturated group on the bioactive moiety. The first reactive unsaturated group may comprise a 1,3-dipole and the second reactive unsaturated group may comprise a dipolarophile. Alternatively, the first reactive unsaturated group may comprise a dipolarophile and the second reactive unsaturated group may comprise a 1,3-dipole. Suitable 1,3-dipoles include, but are not limited to, nitrile oxides, azides, nitrones and nitrile imines. Suitable dipolarophiles include, but are not limited to, alkenes and alkynes. In certain preferred embodiments, the 1,3-dipole comprises an azido group and the dipolarophile comprises an alkyne, *e.g*., ethynyl group. Preferably, the [3+2] cycloaddition is performed in the presence of Cu(I).

In certain embodiments, the toxin moiety in a conjugate of the present invention is selected from the group consisting of anthrax toxin, pertussin toxin, diphtheria toxin, chlorotoxin, botulinum toxin, cholera toxin, *Escherichia coli* heat-labile enterotoxin, pH-sensitive toxins, and functional equivalents thereof. In other embodiments, the toxin moiety is selected from the group consisting of Shiga toxin B-subunit, verotoxin B-subunit, and any functional equivalents thereof.

In certain embodiments, the bioactive moiety in a toxin conjugate of the present invention is a therapeutic moiety. Suitable therapeutic moieties include, but are not limited to, antigens, antigen epitopes, therapeutic antibodies, chemotherapeutics, nucleic acids, and any combinations thereof. In certain preferred embodiments, the therapeutic moiety is an antigen *(e.g.,* a tumor antigen, a viral antigen or a bacterial antigen) or an antigen epitope (e.g., a tumor antigen epitope, a viral antigen epitope or a bacterial antigen epitope). For example, the antigen may be selected from the group consisting of E6, E7, antigens from the Mage family, Her2/neu, EGFRVIII, survivin, telomerase, WT1, ESAT6, Hepatitis B Virus (HBV) antigens L1 and L2, and any active fragments thereof. In certain preferred embodiments, the present invention provides conjugates comprising at least one Shiga toxin B-subunit, or a functional equivalent thereof, and the extracellular domain of Her2/neu.

In other embodiments, the bioactive moiety in a conjugate of the present invention is an imaging moiety. Suitable imaging moieties include, but are not limited to, entities detectable by MRI, entities detectable by MRS, entities detectable by SPECT, and entities detectable by PET.

In another aspect, the present invention provides compositions, in particular pharmaceutical compositions, comprising toxin conjugates disclosed herein. Pharmaceutical compositions of the present invention generally comprise an effective amount of a toxin conjugate described herein and at least one pharmaceutically acceptable carrier or excipient.

In another aspect, the present invention provides medicaments and vaccines comprising an effective amount of a composition comprising a toxin conjugate described herein.

In yet another aspect, the present invention relates to the use of a composition described herein for the manufacture of a medicament or vaccine for the treatment of an antigen-related state in a subject, said antigen-related state being a tumor or an infection. In certain embodiments, the medicament or vaccine is to be administered to a subject in an effective amount for stimulating an immune response against the antigen in the subject, thereby treating the antigen-related state.

In a related aspect, the present invention provides methods for treating an antigen-related state (e.g., a tumor or an infection) in a subject, said methods comprising a step of: administering to the subject an effective amount of a toxin conjugate disclosed herein. The present invention also provides methods for stimulating an immune response in a subject, said methods comprising a step of: administering to the subject a toxin conjugate disclosed herein such that an immune response is stimulated in the subject. In the latter method, an adjuvant may be, optionally administered to the subject in the latter methods.

In another aspect, the present invention provides methods for detecting a tumor that is an antigen-related condition in a subject, said methods comprising steps of: administering to the subject, or contacting a biological obtained from the subject with, an effective amount of a toxin conjugate comprising an imaging moiety as described herein, under conditions to allow the toxin conjugate to interact with any tumor present so that interaction results in binding of the toxin conjugate to the tumor; and detecting any tumor present in the subject or in the biological system and bound to the toxin conjugate using an imaging technique. The biological system may be a cell, a biological fluid or a biological tissue obtained from the subject (e.g., obtained by biopsy). Imaging techniques that can be used in such methods of detection include MRI, MRS, SPECT and PET.

In yet another aspect, the present invention provides methods of preparing toxin conjugates, said methods comprising steps of: providing a toxin moiety comprising a first reactive unsaturated group; and contacting the toxin moiety with a bioactive moiety comprising a second reactive unsaturated group, such that a [3+2] cycloaddition occurs between the first and second unsaturated groups. In such methods, toxin moieties, bioactive moieties, and first and second reactive unsaturated groups are as described above. In certain preferred embodiments, the [3+2] cycloaddition occurs between an azide and an ethynyl group in the presence of Cu(I).

These and other objects, advantages and features of the present invention will become apparent to those of ordinary skill in the art having read the following detailed

### description of the preferred embodiments.

### Brief Description of the Drawing

**Figure 1** presents two schemes showing examples of methods that can be used to covalently attach a toxin moiety to an antigen (or epitope) moiety *via* a [3+2] cycloaddition, according to the present invention.

**Figure 2** presents a scheme illustrating a method according to the present invention for the synthesis of a STxB-BSA hetero-dimer protein. See details of synthesis in Example 1.

**Figure 3** presents results of a Western blot analysis of the Click reaction between STxB-alkyne and BSA-azide carried out as described in Example 1. Lane 1: Click reaction with a 1:1 ratio; Lane 2: Click reaction with a 1:3 ratio; Lane 3: Click reaction in the absence of cupper; Lane 4: BSA alone. The proteins were separated on a 10% Tris-glycine gel.

**Figure 4** presents results of a Western blot analysis of the Click reaction between STxB-alkyne and BSA-azide carried out as described in Example 1. Lane 1: Click reaction in the absence of cupper, Lane 2: Click reaction with a 1:1 ratio; Lane 3: Click reaction with a 1:3 ratio; Lane 4, diluted Click reaction with a 1:1 ratio; Lane 5, molecular weight markers. The proteins were separated on a 10% Tris-glycine gel.

**Figure 5** presents pictures obtained by immunofluorescence. HeLa cells were incubated on ice with 200 nM STxB-BSA. After washing, the cells were shifted to 37°C for 45 minutes, fixed, and double stained for STxB and BSA.

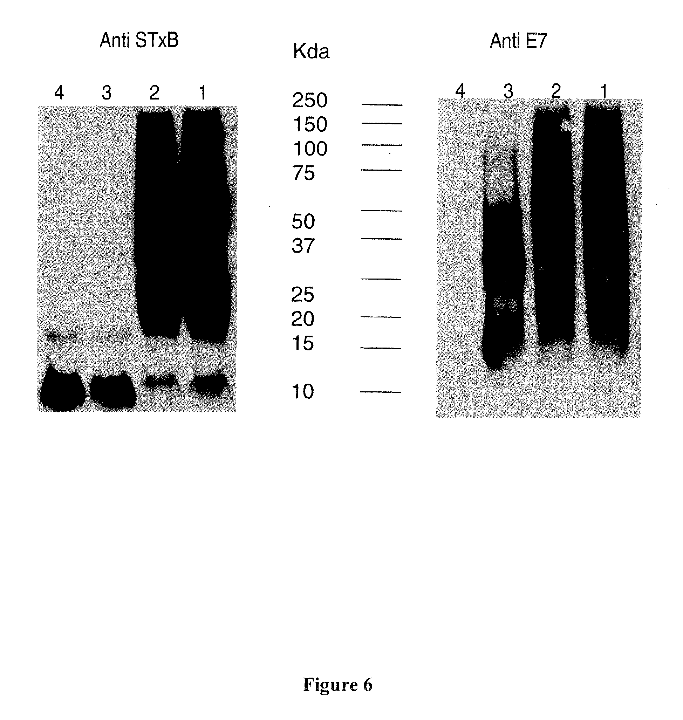

**Figure 6** presents results of a Western blot analysis of the Click reaction between STxB-alkyne and E7-azide carried out as described in Example 2. Lane 1: Click reaction with a 1:9 ratio; Lane 2: Click reaction with a 1:40 ratio; Lane 3, Click reaction in the absence of cupper; Lane 4, StxB alone. The proteins were separated on a 10% Tris-glycine gel.

**Figure 7** presents results of a Western blot analysis of the coupling products between STxB and E7 using the Click Chemistry.

**Figure 8** presents results of a Western blot analysis performed to quantitate the formation of coupling products between STxB and E7 by Click chemistry.

**Figure 9** presents pictures obtained by immunofluorescence. HeLa cells were incubated on ice with 1 µM STxB-E7. After washing, the cells were shifted to 37°C for 45 minutes, fixed, and double stained for STxB and E7.

### Definitions

For purpose of convenience, definitions of a variety of terms used throughout the specification are presented below.

The terms "***protein***", "***polypeptide***", and "***peptide***" are used herein interchangeably, and refer to amino acid sequences of a variety of lengths, either in their neutral (uncharged) forms or as salts, and either unmodified or modified by glycosylation, side chain oxidation, or phosphorylation. In certain embodiments, the amino acid sequence is the full-length native protein. In other embodiments, the amino acid sequence is a smaller fragment of the full-length protein. In still other embodiments, the amino acid sequence is modified by additional substituents attached to the amino acid side chains, such as glycosyl units, lipids, or inorganic ions such as phosphates, as well as modifications relating to chemical conversion of the chains, such as oxidation of sulfhydryl groups. Thus, the term "protein" (or its equivalent terms) is intended to include the amino acid sequence of the full-length native protein, subject to those modifications that do not change its specific properties. In particular, the term "protein" encompasses protein isoforms, *i.e.,* variants that are encoded by the same gene, but that differ in their pI or MW, or both. Such isoforms can differ in their amino acid sequence (*e.g.,* as a result of alternative slicing or limited proteolysis), or in the alternative, may arise from differential post-translational modification (*e*.*g*., glycosylation, acylation or phosphorylation).

The term "***protein analog***", as used herein, refers to a polypeptide that possesses a similar or identical function as a parent polypeptide but need not necessarily comprise an amino acid sequence that is similar or identical to the amino acid sequence of the polypeptide, or possess a structure that is similar or identical to that of the polypeptide. Preferably, in the context of the present invention, a protein analog has an amino acid sequence that is at least about 30%, more preferably at least about 35%, 40%, 45%, 50%, 55%, 60%, 65%, 70%, 75%, 80%, 85%, 90%, 95% or at least 99% identical to the amino acid sequence of the parent polypeptide, and/or contains a characteristic sequence of the parent. Moreover, those of ordinary skill in the art will understand that protein sequences generally tolerate some substitution without destroying activity. Thus, any polypeptide that retains activity and shares at least about 30-40% overall sequence identity, often greater than about 50%, 60%, 70%, or 80%, and further usually including at least one region of much higher identity, often greater than about 90%, 96%, 97%, 98% or 99% in one or more highly conserved regions usually encompassing at least 3-4 and often up to 20 or more amino acids, with the parent polypeptide, is encompassed in the term "protein analog".

The term "***protein fragment***", as used herein, refers to a polypeptide comprising an amino acid sequence of at least 5 amino acid residues of the amino acid sequence of a second polypeptide. The fragment of a protein may or may not possess a functional activity of the full-length native protein.

The term "***biologically active***", when used herein to characterize a protein variant, analog or fragment, refers to a molecule that shares sufficient amino acid sequence identity with the protein to exhibit similar or identical properties than the protein.

The term ***"homologous"*** (or "***homology***"), as used herein, refers to a degree of identity between two polypeptides, molecules or between two nucleic acid molecules. When a position in both compared sequences is occupied by the same base or amino acid monomer subunit, then the respective molecules are homologous at that position. The percentage of homology between two sequences corresponds to the number of matching or homologous positions shared by the two sequences divided by the number of positions compared and multiplied by 100. Generally, a comparison is made when two sequences are aligned to give maximum homology. Homologous amino acid sequences share identical or similar amino acid residues. Similar residues are conservative substitutions for, or "allowed point mutations" of, corresponding amino acid residues in a reference sequence. "Conservative substitutions" of a residue in a reference sequence are substitutions that are physically or functionally similar to the corresponding reference residue, *e.g.,* that have a similar size, shape, electric charge, chemical properties, including the ability to form covalent or hydrogen bonds, or the like. Particularly preferred conservative substitutions are those fulfilling the criteria defined for an "accepted point mutation" by Dayhoff et al. ("Atlas of Protein Sequence and Structure", 1978, Nat. Biomed. Res. Foundation, Washington, DC, Suppl. 3, 22: 354-352).

The term "***isolated***"***,*** when used herein in reference to a protein or polypeptide, means a protein or polypeptide, which by virtue of its origin or manipulation is separated from at least some of the components with which it is naturally associated or with which it is associated when initially obtained. By "isolated", it is alternatively or additionally meant that the protein or polypeptide of interest is produced or synthesized by the hand of man.

As used herein, the term "***reactive unsaturated group***" refers to a functional group containing atoms sharing more than one valence bond and that can undergo addition reactions, in particular cycloadditions. A reactive unsaturated group typically possesses at least one double or triple bond.

As used herein, the term "***cycloaddition***" refers to a chemical reaction in which two or more π-eleetron systems (e.g., unsaturated molecules or unsaturated parts of the same molecule) combine to form a cyclic product in which there is a net reduction of the bond multiplicity. In a cycloaddition, the π electrons are used to form new σ bonds. Products of cycloadditions are called "***adducts***" or "***cycloadducts***". Different types of cycloadditions are known in the art including, but not limited to, [3+2] cycloadditions and Diels-Alder reactions. [3+2] cycloadditions, which are also called 1,3-dipolar cycloadditions, occur between a 1,3-dipole and a dipolarophile and are typically used for the construction of five-membered heterocyclic rings.

The term "***1,3-dipole***" has herein its art understood meaning and refers to a molecule or functional group that is isoelectronic with the allyl anion and has four electrons in a π system encompassing the 1,3-dipole. 1,3-Dipoles generally have one or more resonance structures showing the characteristic 1,3-dipole. Examples of 1,3-dipoles include, but are not limited to, nitrile oxides, azides, diazomethanes, nitrones, and nitrile imines.

As used herein, the term "***dipolarophile***" has its art understood meaning and refers to a molecule or functional group that contains a π bond and that exhibits reactivity toward 1,3-dipoles. The reactivity of dipolarophiles depends both on the substituents present on the π bond and on the nature of the 1,3-dipole involved in the reaction. Dipolarophiles are typically alkenes or alkynes.

As used herein, the term "***click chemistry***"*,* refers to the Huisgen cycloaddition or [3+2] cycloaddition between an azido group and a terminal alkyne group to form a 1,2,4-triazole. Such chemical reactions are generally reliable, selective, and stereospecific.

The terms "***individual***" and "***subject***" are used herein interchangeably. They refer to a human or another mammal (e.g., mouse, rat, rabbit, dog, cat, cattle, swine, sheep, horse or primate) that can suffer from or is susceptible to an antigen-related state or condition but may or may not have the antigen-related state of condition. In many embodiments, the subject is a human being. The terms "individual" and "subject" do not denote a particular age, and thus encompass adults, children, and newborns.

The term "***antigen-related state***" or "***antigen-related condition***"*,* as used herein, refers to micro-organisms or pathogenic infections, allergen-associated states, and diseases and conditions characterized by the presence of tumors (*e*.*g*., breast, ovarian, brain, skin, lung or other types of cancer).

As used herein, the term "***stimulating an immune response***" refers to the initiation of an immune response against an antigen of interest in an individual in which an immune response against said antigen has not already been initiated or refers to any improvement in an immune response that has already been mounted by an individual. It is to be understood that reference to stimulation of an immune response may involve both the humoral and cell-mediated arms of the immune system. Stimulation of an immune response resulting in the stimulation of the humoral immune response may be reflected by an increase in IL-6 which can be determined by any means known in the art, such as for example by ELISA. Stimulation of an immune response resulting in the stimulation of a cell-mediated response may be reflected by an increase in IFN-γ or IL-12, or both, which may be similarly determined. Alternatively or additionally, stimulation of an immune response may be associated with a change in cytokine expression. Such change may be readily measured by any means well-known in the art, such as ELISA, Western Blot analysis, PCR analysis, and others.

The term "***dendritic cells***" (DC), as used herein, refers to antigen-presenting cells, which are capable of presenting antigens to T cells. Dendritic cells include Langerhans cells, interstitial dendritic cells, interdigitating dendritic cells, follicular dendritic cells, and circulating dendritic cells. The term "***mature dendritic cells***"*,* as used herein, refers to a population of dendritic cells with diminished CD115, CD14, CD68 or CD32 expression, or a population of dendritic cells with enhanced CD86 expression, or a combination thereof.

The term "***antigen***"*,* as used herein, refers to any agent (protein, peptide, polysaccharide, glycoprotein, glycolipid, nucleic acid, or combination thereof), which elicits an immune response when introduced into a host. The term "***antigen epitope***" includes fragments of proteins capable of determining antigenicity. An epitope may comprise, for example, a peptide of 6 to 8 residues in length (J. Berzofsky and I Berkower, in "Fundamental Immunology", W. Paul (Ed), 1993, Raven Press: NY, p. 246). However, some epitope may be significantly larger. The affinity of an antibody molecule for its cognate epitope generally ranges from low, *e*.*g*., 10⁻⁶ M, to high, *e.g.,* 10⁻¹¹ M. Antigens include proteins and other molecules which are specifically associated with surfaces of particular types of cancer cells, i.e., tumor cells. The term antigen also refers to any nucleic acid molecule, such as for example DNA, cDNA or RNA, encoding for an antigen or an antigen epitope. Alternatively, antigens may be associated with the surfaces or secretion products of micro-organisms or pathogens. The term "***pathogen***" refers to any organism that causes disorders, such as disorders produced by one or more particular species of bacteria, viruses, fungi, and protozoans, which are disease-producing organisms.

As used herein, the terms "***cancer***" or "***cancerous condition***" refer to or describe a physiological condition in mammals that is typically characterized by unregulated cell growth. Examples of cancers include, but are not limited to, carcinoma, lymphoma, blastoma, sarcoma, and leukemia. More particularly, examples of such cancers include lung cancer, bone cancer, liver cancer, pancreatic cancer, skin cancer, cancer of the head or neck, cutaneous or intraocular melanoma, uterine cancer, ovarian cancer, rectal cancer, cancer of the anal region, stomach cancer, colon cancer, breast cancer, uterine cancer, carcinoma of the sexual and reproductive organs, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the bladder, cancer of the kidney, renal cell carcinoma, carcinoma of the renal pelvis, neoplasms of the central nervous system (CNS), neuroectodermal cancer, spinal axis tumors, glioma, meningioma, and pituitary adenoma.

The term "***treatment***" is used herein to characterize a method or process that is aimed at (1) delaying or preventing the onset of a disease or condition (e.g., an antigen-related state or condition); (2) slowing down or stopping the progression, aggravation, or deterioration of the symptoms of the state or condition; or (3) bringing about ameliorations of the symptoms of the state or condition; or (4) curing the state or condition. A treatment may be administered prior to the onset of the disease, for a prophylactic or preventive action. Alternatively or additionally, a treatment may be administered after initiation of the disease or condition, for a therapeutic action.

The term "***adjuvant***", as used herein, refers to a compound or a composition that may be non-immunogenic when administered in the host alone, but may enhance the host's immune response to an antigen when administered conjointly with the antigen.

The term "***vaccine***"*,* as used herein, refers to a compound or composition that can be used to elicit protective immunity in a recipient. According to the present invention, a vaccine is a medicament.

A "***pharmaceutical composition***" is defined herein as comprising an effective amount of at least one agent of the invention (*i.e.,* a toxin conjugate), and at least one pharmaceutically acceptable carrier or excipient.

As used herein, the term "***effective amount***" refers to any amount of a compound, agent or composition that is sufficient to fulfil its intended purpose(s), *e.g.,* a desired biological or medicinal response in a tissue, system, or subject. For example, in certain embodiments of the present invention, the purpose(s) may be: to stimulate an immune response; to slow down or stop the progression, aggravation, or deterioration of the symptoms of an antigen-related state or condition (*e.g.,* cancer); to bring about amelioration of the symptoms of the disease, to cure the disease; and/or to allow detection and/or diagnostic of an antigen-related state or condition (*e.g.,* tumor).

The term "***pharmaceutically acceptable carrier*** or ***excipient***" refers to a carrier medium which does not interfere with the effectiveness of the biological activity of the active ingredient(s) and which is not excessively toxic to the host at the concentration at which it is administered. The term includes solvents, dispersion media, coatings, antibacterial and antifungal agents, isotonic agents, adsorption delaying agents, and the like. The use of such media and agents for pharmaceutically active substances is well known in the art (see for example, *"*Remington's Pharmaceutical Sciences", E.W. Martin, 18th Ed., 1990, Mack Publishing Co.: Easton, PA, which is incorporated herein by reference in its entirety).

The terms "***therapeutic agent***" and "***drug***" are used herein interchangeably. They refer to a substance, molecule, compound, agent, factor or composition effective in the treatment of a disease or clinical condition.

The term "***diagnostic agent***" refers to a substance, molecule, compound, agent, factor or composition effective in the detection and/or diagnostic of a disease or condition.

The terms "***approximately***" and "***about***"***,*** as used herein in reference to a number, generally include numbers that fall within a range of 10% in either direction of the number (greater than or less than the number) unless otherwise stated or otherwise evident from the context (except where such number would exceed 100% of a possible value).

### Detailed Description of Certain Preferred Embodiments

As mentioned above, the present invention provides methods and compositions for the preparation of toxin conjugates that are useful in vaccination and other clinical applications methods such as, for example, in the diagnostic or treatment of antigen-related states, e.g., tumors and infections. Compared to currently available methods of preparation of toxin conjugates, in particular toxin-antigen conjugates, methods provided by the present invention have the advantage of being simple, reliable, selective, and stereospecific, allowing for the reproducible formation of toxin conjugates.

### I. Preparation of Toxin Conjugates

Methods of preparation of the present invention generally include a chemical reaction between a toxin moiety and a bioactive moiety, wherein the toxin moiety comprises a first reactive group and the bioactive moiety comprises a second reactive group, such that reaction between the first and second reactive groups results in covalent binding of the toxin and bioactive moieties. Preferred reactions are chemically inert toward biomolecules; can take place efficiently under biologically-relevant conditions; and involve reactive groups that are rarely found in naturally-occurring biomolecules. In particular, the present invention provides methods of preparation of toxin conjugates that involve a [3+2] cycloaddition.

### A. [3+2] Cycloaddition

In methods of the present invention, the [3+2] cycloaddition generally occurs between a first reactive unsaturated group on a toxin moiety and a second reactive unsaturated group on a bioactive moiety. In certain preferred embodiments, the second reactive unsaturated group on the bioactive moiety is selected such that it reacts efficiently *via* [3+2] cycloaddition with the first reactive unsatured group on the toxin moiety. More specifically, if the first unsaturated group is a 1,3-dipole, the second unsaturated group will preferably be a dipolarophile that can react with the 1,3-dipole *via* a [3+2] cycloaddition. Alternatively, if the first unsaturated group is a dipolarophile, the second unsaturated group will preferably be a 1,3-dipole that can react with the dipolarophile *via* a [3+2] cycloaddition.

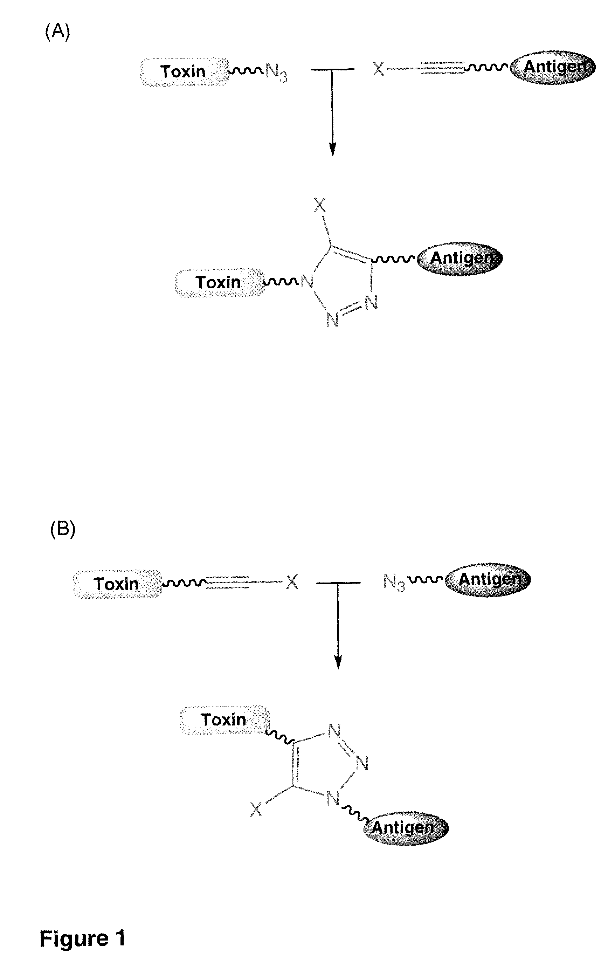

Examples of suitable 1,3-dipoles include, but are not limited to, nitrile oxides, azides, diazomethanes, nitrones, and nitrile imines. Examples of suitable dipolarophiles include, but are not limited to, alkenes (e.g., vinyl, propylenyl, and the like) and alkynes (e.g., ethynyl, propynyl, and the like). In certain embodiments, the 1,3-dipole is an azide, and the dipolarophile is an alkyne, for example, an ethynyl group. Figure 1 shows examples of [3+2] cycloaddition reactions between an azide and an alkyne carried by a toxin moiety and a bioactive moiety (*e.g.,* antigen) which result in the formation of a toxin conjugate according to the present invention. Conjugates formed in these reactions comprise a stable heterocyclic triazol.

In embodiments where the 1,3-dipole is an azide and the dipolarophile is an alkyne, the [3+2] cycloaddition may be performed in the presence of copper(I), which catalyzes the cycloaddition, as described in the art (see, for example, R. Huisgen, Angew. Chem. Int. Ed. Engl, 1963, 2: 565-598; R. Huisgen, Angew. Chem. Int. Ed. Engl., 1963, 2: 633-645; V.V. Rostovtsev et al., Angew Chem., Int. Ed. Engl., 2002, 41: 1596-1599; W.G. Lewis et al., Angew Chem. Int. Ed. Engl., 2002, 41: 1053-1057; C.W. Tornoe et al., J: Org: Chem., 2002, 67: 3057-3064; Q. Wang et al., J. Am. Chem. Soc., 2003, 125: 3192-3193; VD. Block et al., J. Org. Chem., 2006, 51-68). This catalyzed version of [3+2] cycloaddition is termed "Click chemistry".

The Click chemistry has found a plethora of applications (see, for example, Speers et al., J. Am. Chem. Soc., 2005, 127: 10018-10019; Kolb et al., Drug. Discov. Today, 2003, 8, 24, 1128-1137; Deiters et al., J. Am. Chem. Soc., 2003, 125: 11782-11783; Link et al., J. Am. Chem. Soc., 2003, 125: 11164-11165; Wang et al., J. Am. Chem. Soc., 2003, 125: 3192-3193; Agard et al., J. Am. Chem. Soc., 2004, 126: 15046-15047; Link et al., J. Am. Chem. Soc., 2004, 126: 10598-10602; Said Hassane et al., Bioconjug. Chem., 2006, 16: 200-207; and Hatzakis, Chem. Commun., 2006, 2012-2014).

The choice of azides and alkynes as coupling partners is particularly advantageous as they are essentially non-reactive towards each other (in the absence of copper) and are extremely stable and inert towards other chemical groups. It is therefore ideal to ligate two structures featuring a variety of functional groups. This chemical compatibility also helps ensure that many different types of azides and alkynes may be coupled with each other with a minimal amount of side reactions. Furthermore, reaction conditions are mild and suitable for many biomolecules, and no protecting groups are required.

Reaction conditions of the [3+2] cycloaddition will depend on the nature of the toxin and bioactive moieties. Optimization of such reaction conditions is within the skill of the art. Reaction conditions are described, for instance, in Examples 1, 2 and 3, which report the preparation of a STxB/bovine serum albumin (BSA) conjugate, STxB/E7 protein conjugate, and STxB-Her2/neu conjugate, respectively, using a [3+2] cycloaddition according to the present invention.

In certain preferred embodiments, the first reactive unsaturated group and second reactive unsaturated group (*e.g*., 1,3-dipole and dipolarophile) are not found naturally on the toxin moiety and/or bioactive moiety, and have to be introduced. Introduction of such unsaturated groups may be achieved by taking advantage of reactive functional groups present on the toxin moiety and/or bioactive moiety. Alternatively or additionally, reactive functional groups may be added to the toxin moiety and/or bioactive moiety to allow for the introduction of the 1,3-dipole and/or dipolarophile. The term "***reactive functional group***"*,* as used herein, refers to a chemical group that is capable of reacting with another chemical group to form a covalent bond, *i.e.,* that is covalently reactive under suitable reaction conditions. A reactive functional group generally represents a point of attachment for another substance. Reactive functional groups may be selected from a wide variety of chemical groups including, but not limited to, olefins, acetylenes, alcohols, phenols, ethers, oxides, halides, aldehydes, ketones, carboxylic acids, esters, amides, cyanates, isocyanates, thiocyanates, isothiocyanates, amines, hydrazines, hydrazones, hydrazides, diazo, diazonium, nitro, nitriles, mercaptans, sulfides, disulfides, sulfoxides, sulfones, sulfonic acids, sulfinic acids, acetals, ketals, anhydrides, sulfates, sulfenic acids, isonitriles; amidines, imides, imidates, nitrones, hydroxylamines, oximes, hydroxamic acids, thiohydroxamic acids, allenes, ortho esters, sulfites, enamines, ynamines, ureas, pseudoureas, semicarbazides, carbodiimides, carbamates, imines, azides, azo compounds, azoxy compounds, and nitroso compounds. Reactive functional groups also include those usually used to prepare bioconjugates, *e*.*g*., N-hydroxysuccinimide esters, maleimides and the like (see, for example, Hermanson, "Bioconjugate Techniques", Academic Press: San Diego, 1996). Methods to introduce each of these functional groups on proteins or other molecules are well known in the art and their application to or modification for a particular purpose is within the ability of one of skill in the art (see, for example, Sandler and Karo, Eds., "Organic Functional Group Preparations", Academic Press: San Diego, 1989). Reactive functional groups may be protected or unprotected.

### B. Toxin Moieties

Conjugates according to the present invention comprise at least one toxin moiety. As used herein, the term "***toxin moiety***" refers to a toxin or biologically active subunit, variant, derivative or functional equivalent thereof, that specifically binds to a certain type of cells and/or that gets internalized into these cells. A toxin moiety will often exhibit high affinity, selectivity and/or specificity for a certain type of cells, *i.e.,* a toxin moiety will specifically and/or efficiently recognizes, interacts with, binds to, or labels certain cells under the conditions or circumstances of its exposure to the cells. Within a conjugate of the present invention, a toxin moiety confers at least some of its properties to the conjugate, and the conjugate becomes "targeted" to such cells and gets internalized by such cells. Preferably, toxin moieties are stable entities that retain their selectivity/specificity and internalization properties under *in vivo* conditions after conjugation to a bioactive moiety according to the present invention.

Thus, toxin moieties suitable for use in the present invention may be selected among a wide variety of toxins, or functional equivalents thereof, that can be used as delivery systems. For example, a toxin moiety may be selected from the group consisting of anthrax toxin, pertussin toxin, diphtheria toxin, chlorotoxin, botulinum toxin, cholera toxin, *Escherichia coli* heat-labile enterotoxin, pH-sensitive toxins, and functional equivalents thereof.

In many embodiments of the present invention, the toxin moiety is a Shiga toxin B-subunit moiety or a functional equivalent thereof.

Thus, in certain embodiments, the targeting moiety comprises the B-subunit of Shiga toxin, which has the polypeptide sequence described in N.A. Stockbine et al., J. Bacteriol., 1988, 170: 1116-1122, (see also International Patent Publication No. WO 09/03881; European Pat. No. EP 1 386 927; International Patent Publication No. WO 02/060937; and U.S. Pat. No. 6,613,882, International Patent Publication No. WO 04/016148 all by the present Applicants - each of these documents is incorporated herein by reference in its entirety).

In other embodiments, the targeting moiety is a functional equivalent of the Shiga toxin B-subunit. The term "***functional equivalent***", as used herein, means any sequence derived from the B-subunit by mutation, deletion or addition, and with substantially the same routing properties as the B-subunit of the Shiga toxin.

More precisely, a functional equivalent can be constituted by any fragment with the same retrograde transport properties and even intracellular transport to the nucleus as those described for the B-subunit. Examples include, but are not limited to, the B-subunit of verotoxin, described, for example, in S.B. Carderwood et al., Proc. Natl. Acad. Sci. USA, 1987, 84: 4365-4368, and International Publication No. WO 99/59627, and the B-subunit of ricin described, for example, in F.I. Lamb et al., Eur. J. Biochem., 1995, 148: 265-270. After describing the particular transport properties of such fragments, the skilled artisan will be able to select the fragment which would be the best candidate as a vector for routing any therapeutic moiety in any cellular compartment.

The ability of a polypeptidic sequence to bind specifically to the Gb3 receptor may be evaluated using any suitable method. For example, it may be evaluated using an assay based on a method described by Tarrago-Trani (Protein extraction and purification 39, pp. 170-176, 2004), which involves an affinity chromatography on a commercially available galabioase-linked agarose gel (Calbiochem). Galabiose (Galal-4Gal) is the terminal carbohydrate portion of the oligosaccharide moiety of Gb3 and is thought to represent the minimal structure recognized by the B-subunit of Shiga toxin. In such an assay, the protein of interest in PBS buffer (500 µL) is mixed with 100 µL of immobilized galabiose resin previously equilibrated with the same buffer, and incubated for 30 minutes to 1 hour at 4°C on a rotating wheel. After a first centrifugation at 5000 rpm for 1 minute, the pellet is washed twice with PBS. The bound material is then eluted twice by re-suspending the final pellet in 2 x 500 µL of 100 mM glycine pH 2.5. Samples corresponding to the flow-through, the pooled washes and the pooled eluates are then analyzed by SDS Page, Coomassie staining, and Western blotting.

Thus, the present invention encompasses the use of the B-subunit of Shiga toxin or any other subunit or fragment of bacterial toxins which would have activities, in particular routing properties, analogous to those of the B-subunit. This includes polypeptides miming the Shiga toxin B-subunit. These polypeptides, and in general these functional equivalents, can be identified by screening methods which have in common the principle of detecting the interaction between random peptide sequences and the Gb3 receptor or soluble analogues of the receptor. By way of example, phage libraries expressing random peptide sequences for selection on affinity columns comprising Gb3 or after hybridization with soluble radioactive Gb3 analogues can be used.

Other examples of functional equivalents of Shiga toxin B-subunit include those polypeptides containing pre-determined mutations by, *e*.*g*., homologous recombination, site-directed or PCR mutagenesis, and the alleles or other naturally-occurring variants of the family of peptides and derivatives wherein the peptide has been covalently modified by substitution, chemical, enzymatic or other appropriate means with a moiety other than a naturally-occurring amino acid.

Shiga toxin B-subunit moieties and functional equivalents thereof can be prepared using any of a wide variety of methods, including standard solid phase (or solution phase) peptide synthesis methods, as is known in the art. In addition, the nucleic acid encoding these peptides may be synthesized using commercially available oligonucleotide synthesis instrumentation and the proteins may be produced recombinantly using standard recombinant production systems.

As mentioned above, other suitable functional equivalents of the Shiga toxin B-subunit are peptide mimetics that mimic the three-dimensional structure of the naturally-occurring subunit. Such peptide mimetics may have significant advantages over naturally-occurring peptides including, for example, more economical production, greater chemical stability, enhanced pharmacological properties (half-life, absorption, potency, efficacy, etc), altered specificity (e.g., broad-spectrum biological activities, reduced antigenicity and others).

Generally, peptide mimetics are structurally similar to a paradigm polypeptide *(i.e.,* a polypeptide that has a biochemical property or pharmacological activity), but have one or more peptide linkages optionally replaced by a non-peptide linkage. The use of peptide mimetics can be enhanced through the use of combinatorial chemistry to create drug libraries. The design of peptide mimetics can be aided by identifying amino acid mutations that increase or decrease the binding of a peptide to, for example, a tumor cell. Approaches that can be used include the yeast two hybrid method (see, for example, Chien et al., Proc. Natl. Acad. Sci. USA, 1991, 88: 9578-9582) and the phase display method.

In certain embodiments, it may be desirable to chemically modify the Shiga toxin B-subunit moiety (or functional equivalent thereof) to facilitate the introduction of a reactive unsaturated group (*e*.*g*., a 1,3-dipole or a dipolarophile). For example, the present Applicants have designed a Shiga toxin B-subunit (STxB) derivative, or mutant, called STxB-Cys. In this protein, a Cysteine was added at the C-terminus of mature STxB. The protein, when purified from bacteria, carries an internal disulfide bond, as wild type STxB. After modification, the resulting STxB-Cys carries the free sulfhydryl group at the C-terminal Cys (International Publication No. WO 02/060937; M. Amessou et al., "Current Protocols in Cell Biology", Eds. J. Bonifacino et al. (Eds.), Wiley: Hoboken, 2006, Chapter 15.10). Due to their nucleophilicity, free sulfhydryl groups are excellent acceptors for directed coupling approaches. Methods of introducing reactive functional groups on proteins and polypeptides are known in the art.

### C. Bioactive Moieties

Conjugates according to the present invention comprise at least one toxin moiety covalently attached to at least one bioactive moiety through a linker that results from a [3+2] cycloaddition, as disclosed herein. As used herein, the term "***bioactive moiety***" refers to a therapeutic moiety or an imaging moiety.

### C1. Therapeutic Moieties

The term "***therapeutic moiety***"*,* as used herein, refers to any of a wide variety of molecules or biomolecules that are directly or indirectly effective in the treatment or prevention of a disease or clinical condition (*e*.*g*., an antigen-related state or condition). Suitable therapeutic moieties may be selected based on the nature of the toxin moiety and its affinity properties for a certain type of cells. For example, if the toxin moiety within a conjugate of the present invention exhibits a high affinity for cancer cells, suitable therapeutic moieties may be found among anti-cancer agents.

Preferably, therapeutic moieties are stable entities that retain their therapeutic/biological activity when conjugated to a toxin moiety, including *under in vitro* and *in vivo* conditions. Therapeutic moieties may be synthetic or natural compounds. Suitable therapeutic moieties can belong to any of a wide variety of classes of compounds including, but not limited to, small molecules, proteins, peptides, saccharides, steroids, antibodies (including fragments and variants thereof), fusion proteins, antisense polynucleotides, ribozymes, small interfering RNAs, peptidomimetics, and the like.

Due to their selectivity, conjugation methods of the present invention are particularly advantageous when the therapeutic moiety carries a plurality of reactive functional groups that are generally employed in conventional methods of conjugation. Such reactive functional groups include, in particular, amino groups, hydroxyl groups, and thiols. In conventional methods of conjugation, the presence of such reactive functional groups leads to heterogeneous reaction mixtures and batch to batch variability. Thus, in certain preferred embodiments, the therapeutic moiety is selected among molecules or compounds that carry a plurality of reactive functional groups, e.g., more than 1, more than 2, more than 4, more than 6, more than 8 or more than 10 reactive functional groups. Such therapeutic moieties may be found, for example, among antigens or antigen epitopes, therapeutic antibodies, cytokines, growth factors, hormones, chemotherapeutics, nucleic acids, small molecules, and the like.

### Antigens and Epitopes

In embodiments where the toxin moiety is a Shiga toxin B-subunit moiety, or a functional equivalent thereof, conjugates of the present invention may be used for stimulating an immune response. This approach can be envisaged for an anti-infectious or an anti-cancer immunotherapy, or for constituting an antigenic bait in certain autoimmune diseases.

Thus, in certain embodiments of the present invention, the therapeutic moiety comprises an antigen or an antigen epitope. Suitable antigens and antigen epitopes include, but are not limited to, tumor antigens, viral antigens, bacterial antigens, tumor antigen epitopes, viral antigen epitopes, and bacterial antigen epitope. In certain preferred embodiments, the therapeutic moiety comprises a tumor antigen or a tumor antigen epitope.

Tumor antigens include proteins and other molecules which are specifically associated with surfaces of particular types of cancer cells, e.g., tumor cells. Many forms of cancer can be characterized by production of proteins associated with that form of the disease, and are not observed during normal adult lifetime. These antigens are particularly useful as sources of epitopes for anti-cancer vaccines. Tumor antigens suitable for use in the practice of the present invention may be obtained and/or derived from any tissue of the body, including, but not limited to, lung tissue, skin tissue, breast tissue, stomach tissue, colon tissue, rectal tissue, and brain tissue.

For example, breast tumors may be characterized by abnormally expressed receptors, e.g., those of the human-EGF-like receptor family (HER). Additionally, the nestin protein, which is expressed by neuroepithelial stem cells during normal mammalian fetal development, is also expressed on tumors of the central nervous system, including most forms of brain cancer (U.S. Pat. No. 5,338,839). It is also expressed on melanomas, including those that have metastasized to tissues other than the skin (V.A. Florenes et al., Cancer Res., 1994, 54: 354-356). These antigens may be used as therapeutic moieties for the preparation of toxin conjugates according to the present invention.

Other examples of tumors expressing antigens contemplated by the present invention include Wilm's tumor (U.S. Pat. No. 5,350,840), gastrointestinal cancer (WO 95/14085), and cancers characterized by the presence of at least one of a large number of oncogenes well known to the skilled artisan, such as Rb, ras, and c-myc, the sequences of which are available for analysis to those skilled in the art.

Other examples of tumor antigens contemplated as therapeutic moieties in the present invention include MAGE 1 and MAGE 3 or other MAGE antigens (for the treatment of melanoma), PRAME, BAGE, or GAGE (Robbins and Kawakami, Current Opinions in Immunology, 1996, 8:628-636; Van den Eynde et al., Int. J. Clin. Lab. Res., 1997, 27: 81-86; Correale et al., J. Natl. Cancer Inst., 1997, 89: 293-300). These antigens are expressed in a wide range of tumor types such as melanoma, lung carcinoma, sarcoma and bladder carcinoma. Other tumor-specific antigens include, but are not limited to, tumor-specific gangliosides, Prostate specific antigen (PSA) or Her-2/neu, KSA (GA733), PAP, mammaglobin, MUC-1, carcinoembryonic antigen (CEA). Other tumor-associated antigen comprise Prostate-specific membrane antigen (PSMA), Prostate Stem Cell Antigen (PSCA), tyrosinase, survivin, NY-ES01, prostase, PS108 (WO 98/50567), RAGE, LAGE, HAGE. Additionally, said antigen may be a self peptide hormone such as whole length Gonadotrophin hormone releasing hormone (GnRH, WO 95/20600), a short 10 amino acid long peptide, useful in the treatment of many cancers, or in immunocastration.

In certain preferred embodiments of the present invention, the therapeutic moiety comprises an antigen selected from the group consisting of E6, E7, antigens from the Mage family, Her2/neu, EGFRVIII, survivin, telomerase, WT1, ESAT6, Hepatitis B Virus (HBV) antigens L1 and L2.

In a preferred embodiment, the therapeutic moiety comprises a portion or a domain of an antigen. For example, the therapeutic moiety may be the extracellular domain of Her-2/neu, essentially free of transmembrane and cytoplasmic portions, and may be encoded by a cDNA molecule having an amino acid sequence such as defined in Example 3.

Alternatively, therapeutic moieties may be selected among antigens associated with the surfaces or secretion of micro-organisms or pathogens. The term "pathogen" is meant to include organisms that cause disorders, such as disorders produced by one or more particular species of bacteria, viruses, fungi, and protozoans, which are disease-producing organisms. Examples of pathogens include gram-negative bacterial species such as Escherichia coli serotype 0157:H7, Helicobacter pylori, H. mustelae, Haemophilus influenzae and H. ducreyi, Pseudomonas aeruginosa, Shigella dysenteria, Salmonella typhi and S. paratyphi; Gram-positive bacterial species such as Mycobacterium tuberculosis, M leprae, Clostridium tetani, Staphylococcus aureus, and Streptococcus hemolyticus; obligate intracellular bacterial organisms such as Rickettsia and Chlamydia species; retroviruses, which are RNA containing viruses that use reverse transcriptase to synthesize complementary DNA, including but not limited to HIV-1,and -2; other pathogenic viruses such HSV-I and -II, non-A non-B non-C hepatitis virus, pox viruses, and rabies viruses; fungi such as Candida and Aspergillus species; protozoa such as Cryptosporidium parvum, Entamoeba histolytica and Giardia lamblia; and animal pathogens such as Newcastle disease virus. Methods of obtaining unique epitopes from these organisms by screening proteins and by assaying peptides *in vitro* are commonly known to those skilled in the art; many examples have been described and the appropriate amino acid residue sequence may be accessed from Genbank.

Alternatively, therapeutic moieties may be selected among allergens. An allergen is a substance that can induce an allergic or asthmatic response to a susceptible subject. The number of allergens that elicit a sensitive response in a proportion of a population is enormous, and includes pollens, insect venoms, animal dander, dust mite proteins, fungal spores and drugs (*e.g.,* penicillin). Examples of natural animal and plant allergens include proteins specific to the following genera: Felis (Felis domesticus); Canis (Canis familiaris); Dermatophagoides (*e.g.,* Dermatophagoides farinae); Periplaneta (*e.g.,* Periplaneta americana); Ambrosia (Ambrosia artemiisfolia; Lolium (*e.g.,* Lolium perenne or Lolium multiflorum); Cryptomeria (Cryptomeria japonica); Alternaria (Alternaria alternata); Alnus (Alnus gultinosa); Betula (Betula verrucosa); Quercus (Quercus alba); Olea (Olea europa); Artemisia (Artemisia vulgaris); Plantago (*e.g.,* Plantago lanceolata); Parietaria (*e.g.,* Parietaria officinalis or Parietaria judaica); Blattella (*e.g.,* Blattelia germanica); Apis (*e.g.,* Apis multiflorum); Cupressus (*e.g.,* Cupressus sempervirens, Cupressus arizonica and Cupressus macrocarpa); Juniperus (*e.g.,* Juniperus sabinoides, Juniperus virginiana, Juniperus communis and Juniperus ashei); Thuya (*e.g.,* Thuya orientalis), Chamaecyparis (*e.g.,* Chamaecyparis obtusa); Agropyron (*e.g.,* Agropyron repens); Secale (*e.g.,* Secale cereale); Triticum (*e.g.,* Triticum aestivum); Dactylis (*e.g.,* Dactylis glomerata); Festuca (*e.g.,* Festuca elatior); Poa (*e.g.,* Poa pratensis or Poa compressa); Avena (*e.g.,* Avena sativa); Holcus (*e.g.,* Holcus lanatus); Anthoxanthum (*e.g.,* Anthoxanthum odoratum); Arrhenatherum *(e.g.,* Arrhenatherum elatius); Agrostis (*e.g.,* Agrostis alba); Phleum (*e.g.,* Phleum pratense); Phalaris (*e.g.,* Phalaris arundinacea); Paspalum (*e.g.,* Paspalum notatum); Sorghum (*e.g.,* Sorghum halepensis); and Bromus (*e.g.,* Bromus inermis). As used herein, the term "allergen-associated state" refers to conditions which result from an allergic or asthmatic response to an allergen.

In certain embodiments of the present invention, the therapeutic moiety comprises an epitope. Examples of suitable epitopes include, but are not limited to, (a) human epitopes derived from melanoma cell proteins: BAGE from tyrosinase (Boel, P et al (1995), Immunite 2, 167-75); GAGE from gp75 (Van den Eynde, B. et al (1995), J. Exp. Med. 182, 689-98); tyrosinase (Brichard V. et al (1993), J. Exp. Med. 178, 489-95); p15 from A/MART-1 melanoma (Coulie P. G. et al (1994), J. Exp. Med. 180, 35-42; Kawakami Y. et al (1994), J. Exp. Med. 180, 347-52); MAGE-1 and -3 from β-catenin (De Plaen E. et al (1994), Immunogenetics 40, 369-9; Traversari C et al (1992), J. Exp. Med. 176, 1453-7.); (b) Human epitopes derived from virus proteins involved in cancer development; Peptides derived from E6 and E7 proteins of HPV 16 (Feltkamp M. C. et al (1993), Eur. J. Immunol. 23, 2242-9; Davis H. L. et al (1995), Hum. Gene Ther. 6, 1447-56); Peptides derived from the Hbs protein of HBV (Rehermann B. et al (1995), J. Exp. Med. 181, 1047-58); Peptides derived from proteins from EBV (Murray R. J. et al (1992), J. Exp. Med. 176, 157-68); Peptides derived from cytomegalovirus; (c) human epitopes derived from oncogenes: p21ras (Peace D. J. (1993), J. Immunother 14, 1104; Ciemik, I. F. et al (1995) Hybridoma 14, 139-42); p53 (Gnjatic S. (1995), Eur. J. Immunol. 25, 1638-42); (d) epitopes of interest in autoimmune diseases, and (e) epitopes of interest in infectious diseases. Examples of such epitopes are, for example, described in K. Furukawa et al., J. Clin. Invest., 1994, 94: 1830-1839.

The list of antigens, antigen epitopes, and allergens provided above represents only a very small number of antigens, epitopes and allergens that can be used as therapeutic moieties for the preparation of conjugates according to the present invention. Selection of an antigen, epitope or allergen as therapeutic moiety for a given therapeutic use of an inventive conjugate is within the competence of one skilled in the art.

### Therapeutic Antibodies

In certain embodiments, the therapeutic moiety covalently linked to the toxin moiety, e.g., the Shiga toxin B-subunit moiety, or a functional equivalent thereof, in a conjugate of the present invention is an antibody with therapeutic activity.

Examples of antibodies that may be used in the present invention include monoclonal antibodies (mAbs), for example, chimeric antibodies, humanized antibodies, primatized antibodies, resurfaced antibodies, human antibodies and biologically active fragments thereof. The term antibody is used broadly to refer to both antibody molecules and a variety of antibody-derived molecules. Such antibody-derived molecules generally comprise at least one complementarity determining region (CDR) from either a heavy chain or light chain variable region, including molecules such as Fab fragments, F(ab')₂ fragments, Fd fragments, Fabc fragments, Sc antibodies (single chain antibodies), diabodies, individual antibody light single chains, individual antibody heavy chains, chimeric fusions between antibody chains and other molecules, and the like. Antibodies mimetics having binding affinity for an antigen but not having one or more traditional CDRs can also be used in the formation of conjugates of in the present invention.

For example, antibodies that can be used as therapeutic moieties may be antibodies that are useful in the treatment of cancer. Examples of such antibodies include, but are not limited to, alemtuzmab, antibodies against prostate-specific membrane antigen (such as MLN-591, MLN591RL and MLN2704), Avastin^{™} (bevacizumab), (or other anti-VEGF antibody), Rituximab (RITUXAN^{™}), Trastuzumab (Herceptin^{™} or other anti-Her2 antibody), 2C4 (or other antibody which interferes with HER2-mediated signaling), and the like.

### Anti-Cancer Drugs

In certain embodiments, the therapeutic moiety covalently linked to a toxin moiety, *e*.*g*., the Shiga toxin B-subunit moiety, or a functional equivalent thereof, in a conjugate of the present invention is an anti-cancer drug. Suitable anti-cancer drugs for use as therapeutic moieties in the present invention can be found, for example, among any of a variety of anti-cancer drugs including, but not limited to, chemotherapeutic agents, such as alkylating agents, purine antagonists, pyrimidine antagonists, intercalating antibiotics, aromatase inhibitors, growth factor inhibitors, cell cycle inhibitors, enzymes, topoisomerase inhibitors, biological response modifiers, intercalating agents, topoisomerase I inhibitors, antimetabolite drugs, anti-mitotic antibiotics, alkaloidal anti-tumor agents, hormones and anti-hormones, interferon, non-steroidal anti-inflammatory drugs, and various other anti-tumor agents such as kinase inhibitors, proteasome inhibitors, and NF-κB inhibitors.

Chemotherapeutics may also be selected among pro-apoptotic agents. In certain embodiments, pro-apoptotic agents are ligands to mitochondrial peripheral benzodiazepine receptor (mPBR). For therapeutic intervention, it is necessary to find specific ligands that do not interfere with the benzodiazepine receptors of the central nervous system.

In certain embodiments, the therapeutic moiety is the chemotherapeutic SN38 or the chemotherapeutic RO5-4864

### D. Other sequences

In addition to a toxin moiety and a therapeutic moiety, conjugates of the present invention may further comprise any sequence necessary for maturation of the protein in a suitable cellular system, and/or any sequence necessary for recognition of a given cell type by the conjugate, thus enabling selectivity of action and penetration into the cell cytoplasm.

The term "maturation", as used herein, refers to any process which, from a given polypeptide, leads to the emergence of peptides which themselves can be presented in a cellular compartment including the cytoplasm. Maturation can occur either by enzymatic clipping in the endoplasmic reticulum, or by transport into the cytoplasm in which the polypeptide is cleaved then the peptides obtained are again transported in the endoplasmic reticulum. Molecules of the class I major histocompatibility complex (cl I MHC) can become charged with polypeptide molecules of interest after such cleavage and be presented on the cellular membranes.

Thus, in certain embodiments, a conjugate of the present invention may comprise, in addition to a toxin moiety covalently linked to a therapeutic moiety, (a) modifications sites such as an N-glycosylation site constituted, for example, by about 20 amino acids; phosphorylation sites or any sequence necessary for any maturation of the conjugate; (b) a retention signal of the tetrapeptide KDEL type (Lys-Asp-Glu-Leu), which, when it is bound to a carboxy-terminal end of resident ER proteins, causes retention after maturation of the proteins by passage into the Golgi apparatus. For details about the role of a retention signal in protein maturation, see, for example, M.J. Lewis et al., Cell, 1992, 68: 353-364.

### C2. Imaging Moieties

In certain embodiments, bioactive moieties are imaging moieties. In the context of the present invention, imaging moieties are entities that are detectable by imaging techniques such as Magnetic Resonance Imaging (MRI), Magnetic Resonance Spectroscopy (MRS), Single Photon Emission Computed Tomography (SPECT) or Positron Emission Tomography (PET). Preferably, imaging moieties are stable, non-toxic entities that retain their properties under *in vitro* and *in vivo* conditions.

*MRI Imaging Moieties.* In certain embodiments, conjugates of the present invention are designed to be detectable by Magnetic Resonance Imaging. MRI has evolved into one of the most powerful non-invasive techniques in diagnostic clinical medicine and biomedical research (P. Caravan et al., Chem. Rev. 1999, 99: 2293-2352; W. Khun, Angew. Chem. Int. Ed. Engl. 1990, 29: 1-19; M.M. Huber et al., Bioconjug. Chem. 1998, 9: 242-249; R.A. Moats et al., Angew. Chem. Int. Ed. Engl. 1997, 36: 726-728; X. Yu et al., Mag. Res. Med. 2000, 44: 867-872). MRI is an application of Nuclear Magnetic Resonance (NMR), a well known analytical method used in chemistry, physics and molecular structural biology. MRI can generate three dimensional structural information in relatively short time spans and is widely used as a non-invasive diagnostic tool to identify potentially maleficent physiological anomalies, to observe blood flow or to determine the general status of the cardiovascular system (P. Caravan et al., Chem. Rev. 1999, 99: 2293-2352).

Most MRI contrast agents typically consist of chelated paramagnetic metal ions. Thus, in certain embodiments, imaging moieties comprise at least one metal-chelating moiety complexed to a paramagnetic metal ion.

Suitable paramagnetic metal ions include any of the paramagnetic metal ions known to be physiologically acceptable, good contrast enhancers in MRI, and easily incorporated into metal-chelating moieties. Preferably, the paramagnetic metal ion is selected from the group consisting of gadolinium III (Gd³⁺), chromium III (Cr³⁺), dysprosium III (Dy³⁺), iron III (Fe³⁺), manganese II (Mn²⁺), and ytterbium III (Yb³⁺). More preferably, the paramagnetic metal ion is gadolinium III (Gd³⁺). Gadolinium is an FDA-approved contrast agent for MRI, which accumulates in abnormal tissues causing these abnormal areas to become very bright (enhanced) on the MRI. Gadolinium is known to provide great contrast between normal and abnormal tissues in different areas of the body, in particular in the brain.

Suitable metal-chelating moieties include any of the entities known in the art to complex paramagnetic metal ions detectable by MRI. Preferably, a metal-chelating moiety is a stable, non-toxic entity that binds a paramagnetic metal ion in such a way that it leaves one coordination site open for a water molecule and with such high affinity that, once complexed, the paramagnetic metal ion cannot be displaced by water. Examples of metal-chelating moieties include, but are not limited to, DTPA; DOTA and derivatives thereof (see, for example, U.S. Pat. Nos. 4,885,363; 5,087,440; 5,155,215; 5,188,816; 5,219,553; 5,262,532; and 5,358,704; and D. Meyer et al., Invest. Radiol. 1990, 25: S53-55 and DTPA-bis(amide) derivatives (U.S. Pat. No. 4,687,659). Other metal-chelating moieties that complex paramagnetic metal ions include acyclic entities such as aminopolycarboxylic acids and phosphorus oxyacid analogues thereof (*e.g.,* triethylenetetraminehexaacetic acid or TTHA, and dipyridoxal diphosphate, DPDP) and macrocyclic entities (*e.g.,* DO3A). Metal-chelating moieties may also be any of the entities described in U.S. Pat. Nos. 5,410,043; 5,277,895; and 6,150,376; or in F.H. Arnold, Biotechnol. 1991, 9: 151-156.

*MRS Imaging Moieties.* In certain embodiments, conjugates of the present invention are designed to be detectable by Magnetic Resonance Spectroscopy (MRS). More specifically, the present invention also provides conjugates comprising at least one toxin moiety linked to at least one entity (*e.g.,* a therapeutic moiety) labeled with a stable paramagnetic isotope, such as carbon-13 (¹³C) and fluorine-19 (¹⁹F).

*Radioactive Imaging Moieties.* In other embodiments, conjugates of the present invention are designed to be detectable by Single Photon Emission Computed Tomography (SPECT) or Positron Emission Tomography (PET).

Preferably, imaging moieties comprise at least one metal-chelating moiety complexed to a metal entity that is detectable by SPECT or PET. SPECT is similar to PET, but the radioactive substances used in SPECT have longer decay times than those used in PET and emit single instead of double gamma rays. Suitable metal entities for use in the present invention are radionuclides known in the art to be physiologically acceptable, detectable by SPECT or PET, and easily incorporated into metal-chelating moieties. Preferably, the radionuclide is selected from the group consisting of technetium-99m (^{99m}Tc), gallium-67 (⁶⁷Ga), yttrium-91 (⁹¹Y), indium-111 (¹¹¹In), rhenium-186 (¹⁸⁶Re), and thallium-201 (²⁰¹Tl). Most preferably, the radionuclide is technetium-99m (^{99m}Tc). Over 85% of the routine nuclear medicine procedures that are currently performed use radiopharmaceutical methodologies based on ^{99m}Tc.

Suitable metal-chelating moieties for use in the present invention include any of the entities known to complex short-lived radionuclides detectable by SPECT or PET. Preferably, metal-chelating moieties are stable, non-toxic entities that bind radionuclides detectable by SPECT or PET with high affinity. Metal-chelating moieties that complex radionuclides such as ^{99m}Tc are well known in the art (see, for example, Technetium and Rhenium in Chemistry and Nuclear Medicine", M. Nicolini et al., Eds., 1995, SGEditoriali: Padova, Italy). Suitable metal-chelating moieties include, for example, N₂S₂ and N₃S chelators (A.R. Fritzberg et al., J. Nucl. Med. 1982, 23: 592-598) which can complex a radionuclide through two nitrogen atoms and two sulfur atoms, or through three nitrogen atoms and one sulfur atom, respectively. Ethyl cysteine dimer (ECD) is an N₂S₂ chelator well known in the art. N₂S₂ and N₃S chelators are, for example, described in U.S. Pat. Nos. 4,444,690; 4,670,545; 4,673,562; 4,897,255; 4,965,392; 4,980,147; 4,988,496; 5,021,556 and 5,075,099.

Other suitable metal-chelating moieties can be selected from polyphosphates (e.g., EDTMP); aminocarboxylic acids (e.g., EDTA); 1,3-diketones; hydroxycarboxylic acids; polyamines; aminoalcohols; aromatic heterocyclic bases; phenols; aminophenols; oximes; Schiff bases; tetrapyrroles; sulfur compounds; synthetic macrocyclic compounds (e.g., dibenzo[18]crown-6), or combinations of two or more of the above agents. Other suitable metal-chelating moieties are described in U.S. Pat. No. 5,559,214, and in WO 95/26754, WO 94/09056, WO 94/29333, WO 94/08624, WO 94/08629, WO 94/13327, and WO 94/12216.

### II. Pharmaceutical Compositions and Formulations

Conjugates described herein may be administered *per se* or in the form of a pharmaceutical composition. Accordingly, the present invention provides pharmaceutical compositions comprising an effective amount of at least one inventive toxin conjugate and at least one pharmaceutically acceptable carrier, vehicle or excipient.

Pharmaceutical compositions according to the present invention may be administered using any amount and any route of administration effective for achieving the desired effect. The exact amount of pharmaceutical composition to be administered will vary from subject to subject, depending on the species, age, and general condition of the subject, the severity of the condition, and the like (see below).

The optimal pharmaceutical formulation can be varied depending upon the route of administration and desired dosage. Such formulations may influence the physical state, stability, rate of *in vivo* release, and rate of *in vivo* clearance of the administered compounds.

Pharmaceutical compositions of the present invention may be formulated in dosage unit form for ease of administration and uniformity of dosage. The expression "***unit dosage form***"*,* as used herein, refers to a physically discrete unit of conjugate (with or without one or more additional agents) for the patient to be treated. It will be understood, however, that the total daily usage of compositions of the present invention will be decided by the attending physician within the scope of sound medical judgment.

After formulation with one or more appropriate pharmaceutically acceptable carrier(s) or excipient(s) in a desired dosage, pharmaceutical compositions of the present invention can be administered to humans or other mammals by any suitable route. Methods of administration include, but are not limited to, dermal, intradermal, intramuscular, intraperitoneal, intravenous, subcutaneous, intranasal, pulmonary, epidural, ocular, and oral routes. An inventive composition may be administered by any convenient or otherwise appropriate route, for example, by infusion or bolus injection, by absorption through epithelial or mucocutaneous linings (e.g., oral, mucosa, rectal and intestinal mucosa, etc) and may be administered together with other biologically active agents. Administration can be systemic or local. In embodiments where a pharmaceutical composition of the present invention is to be used as a vaccine, the pharmaceutical composition is preferably administered orally, transdermally or intrabronchially.

Pharmaceutical compositions of the present invention may be prepared according to general pharmaceutical practice (see, for example, *"*Remington's Pharmaceutical Sciences", E.W. Martin, 18th Ed., 1990, Mack Publishing Co.: Easton, PA and *"*Encyclopedia of Pharmaceutical Technology", 1988, J. Swarbrick, and J.C. Boylan (Eds.), Marcel Dekker, Inc: New York, each of which is incorporated herein by reference in its entirety).

Injectable preparations, for example, sterile injectable aqueous or oleaginous suspensions may be formulated according to the known art using suitable dispersing or wetting agents, and suspending agents. A sterile injectable preparation may also be a sterile injectable solution, suspension or emulsion in a non-toxic parenterally acceptable diluent or solvent, for example, as a solution in 2,3-butanediol. Among the acceptable vehicles and solvents that may be employed are water, Ringer's solution, U.S.P. and isotonic sodium chloride solution. In addition, sterile, fixed oils are conventionally employed as a solution or suspending medium. For this purpose any bland fixed oil can be employed including synthetic mono- or di-glycerides. Fatty acids such as oleic acid may also be used in the preparation of injectable formulations. Sterile liquid carriers are useful in sterile liquid from compositions for parenteral administration.

Injectable formulations can be sterilized, for example, by filtration through a bacterial-retaining filter, or by incorporation of sterilizing agents in the form of sterile solid compositions which can be dissolved or dispersed in sterile water or other sterile injectable medium prior to use. Liquid pharmaceutical compositions which are sterile solutions or suspensions can be administered by, for example, intravenous, intramuscular, intraperitoneal or subcutaneous injection. Injection may be *via* single push or by gradual infusion (*e*.*g*., 30 minute intravenous infusion). Where necessary, the composition may include a local anesthetic to ease pain at the site of injection.

In order to prolong the effect of a drug (*e*.*g*., toxin conjugate), it is often desirable to slow the absorption of the drug from subcutaneous or intramuscular injection. This may be accomplished by the use of a liquid suspension of crystalline or amorphous material with poor water solubility. The rate of absorption of the drug then depends upon its rate of dissolution which, in turn, may depend upon crystal size and crystalline form. Alternatively, delayed absorption of a parenterally administered drug form is accomplished by dissolving or suspending the drug in an oil vehicle. Injectable depot forms are made by forming micro-encapsuled matrices of the drug in biodegradable polymers such as polylactide-polyglycolide. Depending upon the ratio of drug to polymer and the nature of the particular polymer employed, the rate of drug release can be controlled. Examples of other biodegradable polymers include poly(orthoesters) and poly(anhydrides). Depot injectable formulations can also be prepared by entrapping the drug in liposomes or microemulsions which are compatible with body tissues.

Liquid dosage forms for oral administration include, but are not limited to, pharmaceutically acceptable emulsions, microemulsions, solutions, suspensions, syrups, elixirs, and pressurized compositions. In addition to the active ingredient (*i*.*e*., toxin conjugate), the liquid dosage form may contain inert diluents commonly used in the art such as, for example, water or other solvent, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cotton seed, ground nut, corn, germ, olive, castor, and sesame oils), glycerol, tetrahydrofurfuryl alcohol, polyethylene glycols and fatty acid esters of sorbitan, and mixtures thereof. Besides inert diluents, oral compositions can also include adjuvants such as wetting agents, suspending agents, preservatives, sweetening, flavoring, and perfuming agents, thickening agents, colors, viscosity regulators, stabilizers or osmo-regulators. Suitable examples of liquid carriers for oral administration include water (partially containing additives as above; *e*.*g*., cellulose derivatives, such as sodium caboxymethyl cellulose solution), alcohols (including monohydric alcohols and polyhydric alcohols such as glycols) and their derivatives, and oils (*e.g.,* fractionated coconut oil and arachis oil)).

Solid dosage forms for oral administration include, for example, capsules, tablets, pills, powders, and granules. In such solid dosage forms, the active ingredient is mixed with at least one inert, physiologically acceptable excipient or carrier such as sodium citrate or dicalcium phosphate and one or more of: (a) fillers or extenders such as starches, lactose, sucrose, glucose, mannitol, and silicic acid; (b) binders such as, for example, carboxymethylcellulose, alginates, gelatin, polyvinylpyrrolidone, sucrose, and acacia; (c) humectants such as glycerol; (d) disintegrating agents such as agar-agar, calcium carbonate, potato or tapioca starch, alginic acid, certain silicates, and sodium carbonate; (e) solution retarding agents such as paraffin; (f) absorption accelerators such as quaternary ammonium compounds; (g) wetting agents such as, for example, cetyl alcohol and glycerol monostearate; (h) absorbents such as kaolin and bentonite clay; and (i) lubricants such as talc, calcium stearate, magnesium stearate, solid polyethylene glycols, sodium lauryl sulfate, and mixtures thereof. Other excipients suitable for solid formulations include surface modifying agents such as non-ionic and anionic surface modifying agents. Representative examples of surface modifying agents include, but are not limited to, poloxamer 188, benzalkonium chloride, calcium stearate, cetostearyl alcohol, cetomacrogol emulsifying wax, sorbitan esters, colloidal silicon dioxide, phosphates, sodium dodecylsulfate, magnesium aluminum silicate, and triethanolamine. In the case of capsules, tablets and pills, the dosage form may also comprise buffering agents. The amount of solid carrier per solid dosage form will vary widely but preferably will be from about 25 mg to about 1 g.

Solid compositions of a similar type may also be employed as fillers in soft and hard-filled gelatin capsules using such excipients as lactose or milk sugar as well as high molecular weight polyethylene glycols and the like. The solid dosage forms of tablets, dragées, capsules, pills, and granules can be prepared with coatings and shells such as enteric coatings, release controlling coatings and other coatings well known in the pharmaceutical formulating art. They may optionally contain opacifying agents and can also be of a composition such that they release the active ingredient(s) only, or preferentially, in a certain part of the intestinal tract, optionally, in a delayed manner. Examples of embedding compositions which can be used include polymeric substances and waxes.

In certain embodiments, it may be desirable to administer an inventive composition locally to an area in need of treatment. This may be achieved, for example, and not by way of limitation, by local infusion during surgery, topically application, by injection, by means of a catheter, by means of suppository, or by means of a skin patch or stent or other implant.

For topical administration, a composition is preferably formulated as a gel, an ointment, a lotion, or a cream which can include carriers such as water, glycerol, alcohol, propylene glycol, fatty alcohols, triglycerides, fatty acid esters, or mineral oil. Other topical carriers include liquid petroleum, isopropyl palmitate, polyethylene glycol, ethanol (95%), polyoxyethylenemonolaurate (5%) in water, or sodium lauryl sulfate (5%) in water. Other materials such as antioxidants, humectants, viscosity stabilizers, and similar agents may be added as necessary. Percutaneous penetration enhancers such as Azone may also be included.

In addition, in certain instances, it is expected that inventive compositions may be disposed within transdermal devices placed upon, in, or under the skin. Such devices include patches, implants, and injections which release the compound onto the skin, by either passive or active release mechanisms. Transdermal administrations include all administrations across the surface of the body and the inner linings of bodily passage including epithelial and mucosal tissues. Such administrations may be carried out using the present compositions in lotions, creams, foams, patches, suspensions, solutions, and suppositories (rectal and vaginal).

Transdermal administration may be accomplished through the use of a transdermal patch containing active ingredient(s) and a carrier that is non-toxic to the skin, and allows the delivery of at least some of the active ingredient(s) for systemic absorption into the bloodstream *via* the skin. The carrier may take any number of forms such as creams and ointments, pastes, gels, and occlusive devices. Creams and ointments may be viscous liquid or semisolid emulsions of either the oil-in-water or water-in-oil type. Pastes comprised of absorptive powders dispersed in petroleum or hydrophilic petroleum containing active ingredient(s) may also be suitable. A variety of occlusive devices may be used to release active ingredient(s) into the bloodstream such as a semi-permeable membrane covering a reservoir containing the active ingredient(s) with or without a carrier, or a matrix containing the active ingredient.

Suppository formulations may be made from traditional materials, including cocoa butter, with or without the addition of waxes to alter the suppository's melting point, and glycerin. Water soluble suppository bases, such as polyethylene glycols of various molecular weights, may also be used.

For administration by inhalation, an inventive toxin conjugate may be delivered in the form of an aerosol spray from pressured container or dispenser which contains a suitable propellant, *e*.*g*., a gas such as carbon dioxide or a nebulizer.

Materials and methods for producing various formulations are known in the art and may be adapted for practicing the subject invention.

In certain embodiments, a pharmaceutical composition further comprises at least one adjuvant. As defined previously, an "adjuvant" is a substance that serves to enhance the immunogenicity of an antigen. Thus, adjuvants are often given to boost the immune response and are well known in the art. Examples of adjuvants suitable for use in the present invention include, but are not limited to, saponin lipid A or a derivative thereof, an immunostimulatory oligonucleotide such as CpG containing oligonucleotides, an alkylglucosaminide phosphate, a Toll like agonist such as 3D-MLP, Qs21 or alkyl glucosaminide phosphates (TLR4 agonist), peptidoglycan or lipoprotein (TLR2 agonist), imidazoquinolines (TLR7 agonist), single stranded RNA (TLR8 agonist), or double-stranded RNA, poly IC (TLR3 agonist), ligands of CDI able to stimulate NKT cells, and in a preferred embodiment, a glycolipid, a phospholipid, a glucosphingo lipid, a derivative or an analog thereof, and in a more preferred embodiment, a glycosylceramide.

Compositions of the present invention may be used for the fabrication of a medicament for the treatment of an antigen-related state. Medicaments include vaccines and diagnostic agents. Thus, in another aspect, the present invention also provides medicaments comprising an effective amount of a composition of a toxin conjugate described herein.

### III. Dosages and Administration

A treatment according to the present invention may consist of a single dose or a plurality of doses over a period of time.

Administration may be one or multiple times daily, weekly (or at some other multiple day interval) or on an intermittent schedule. For example, an inventive pharmaceutical composition may be administered one or more times per day on a weekly basis for a period of weeks (*e.g.,* 4-10 weeks). Alternatively, an inventive pharmaceutical composition may be administered daily for a period of days (*e*.*g*., 1-10 days) following by a period of days (*e*.*g*., 1-30 days) without administration, with that cycle repeated a given number of times (*e*.*g*., 2-10 cycles).

Administration may be carried out in any convenient manner such as by injection (subcutaneous, intravenous, intramuscular, intraperitoneal, or the like) or oral administration.

Depending on the route of administration, effective doses may be calculated according to the body weight, body surface area, or organ size of the subject to be treated. Optimization of the appropriate dosages can readily be made by one skilled in the art in light of pharmacokinetic data observed in human clinical trials. Final dosage regimen will be determined by the attending physician, considering various factors which modify the action of the drugs, e.g., the drug's specific activity, the severity of the damage and the responsiveness of the patient, the age, condition, body weight, sex and diet of the patient, the severity of any present infection, time of administration, the use (or not) of concomitant therapies, and other clinical factors. As studies are conducted using the inventive combinations, further information will emerge regarding the appropriate dosage levels and duration of treatment.

Typical dosages comprise 1.0 pg/kg body weight to 100 mg/kg body weight. For example, for systemic administration, dosages may be 100.0 ng/kg body weight to 10.0 mg/kg body weight. For direct administration to the site *via* microinfusion, dosages may be 1 ng/kg body weight to 1 mg/kg body weight.

In embodiments where the pharmaceutical composition is used as a vaccine. The amount of antigen in each vaccine will preferably be an amount which induces an immunoprotective response without significant adverse side effects. Generally, it is expected that each human dose of vaccine will comprise 0.1-1000 µg of antigen, preferably 0.1-500 µg, preferably 0.1-100 µg, most preferably 0.1 to 50 µg. An optimal amount for a particular vaccine can be ascertained by standard studies involving observation of appropriate immune responses in vaccinated subjects. Following an initial vaccination, subjects may receive one or several booster immunisations adequately spaced. Such a vaccine formulation may be applied to a mucosal surface of a mammal in either a priming or a boosting vaccination regime; or alternatively be administered systemically, for example *via* the transdermal, subcutaneous or intramuscular routes. In certain embodiments, intramuscular administration is preferred.

It will be appreciated that pharmaceutical combinations of the present invention can be employed in combination with additional therapies (*i.e.,* a treatment according to the present invention can be administered concurrently with, prior to, or subsequently to one or more desired therapeutics or medical procedures). The particular combination of therapies (therapeutics or procedures) to employ in such a combination regimen will take into account compatibility of the desired therapeutics and/or procedures and the desired therapeutic effect to be achieved.

### IV. Indications

In another aspect, the present invention relates to the use of an inventive toxin conjugate, or a composition thereof, for treating an antigen-related state or condition in a subject in need thereof. The present invention also relates to the use of an inventive conjugate, or a composition thereof, for stimulating an immune response in a subject in need thereof.

More specifically, the present invention provides methods for treating an antigen-related state in a subject, said methods comprising a step of administering, to the subject, an effective amount of a toxin conjugate, or composition thereof, described herein. As already mentioned above, antigen-related states include, for example, infections and clinical conditions characterized by the presence of tumors.

Thus, in certain embodiments, compositions and methods of the present invention are used for treating an infection, such as for example, a micro-organism or pathogenic infection. The term "infection", as used herein, has its art understood meaning and is meant to include persistence and growth of a pathogen in a subject host. While symptoms used to diagnose the presence of infection include fever, inflammation, pain, joint and muscular sensations at or near the sited of infection, the absence of one or more of these symptoms do not preclude infection in a subject host organism. The term "inflammation" indicates a set of host reactions that accompany infection, and may also be present in the absence of infection, for example, as a symptom of autoimmune reactions, degenerative diseases, tissue remodelling disorders, exposure to allergens, and/or other conditions. Inflammatory responses include cellular processes, such as neutrophil mast cell and basophil degranulation with associated release of proteases, histamines, and superoxide generation, and production of and responses to cytokines such as interferons and humor necrosis factor.

Infections that can be treated by compositions and methods of the present invention include, but are not limited to, HIV (Human Immunodeficiency Virus) infection, HPV (Human papillomavirus) infection, HCV (hepatitis C) infection, HBV (hepatitis B) infection, malaria, and tuberculosis infection (TB).

In other embodiments, compositions and methods of the present invention are used for treating a disease or condition characterized by the presence of tumors, *e*.*g*., in the treatment of primary and/or metastatic cancers and other cancerous conditions. For example, inventive compositions and methods may be useful for reducing the size of solid tumors, inhibiting tumor growth or metastasis, and/or prolonging the survival of mammals (including humans) suffering from diseases.

Examples of cancers and cancer conditions that can be treated according to the present invention include, but are not limited to, tumors of the brain and central nervous system (*e.g.,* tumors of the meninges, brain, spinal cord, cranial nerves and other parts of the CNS, such as glioblastomas or medulla blastomas); head and/or neck cancer, breast tumors, tumors of the circulatory system (*e.g.,* heart, mediastinum and pleura, and other intrathoracic organs, vascular tumors, and tumor-associated vascular tissue); tumors of the blood and lymphatic system (*e.g.,* Hodgkin's disease, Non-Hodgkin's disease lymphoma, Burkitt's lymphoma, AIDS-related lymphomas, malignant immunoproliferative diseases, multiple myeloma, and malignant plasma cell neoplasms, lymphoid leukemia, myeloid leukemia, acute or chronic lymphocytic leukemia, monocytic leukemia, other leukemias of specific cell type, leukemia of unspecified cell type, unspecified malignant neoplasms of lymphoid, haematopoietic and related tissues, such as diffuse large cell lymphoma, T-cell lymphoma or cutaneous T-cell lymphoma); tumors of the excretory system (*e.g.,* kidney, renal pelvis, ureter, bladder, and other urinary organs); tumors of the gastronintestinal tract (*e.g.,* oesophagus, stomach, small intestine, colon, colorectal, rectosigmoid junction, rectum, anus, and anal canal); tumors involving the liver and intrahepatic bile ducts, gall bladder, and other parts of the biliary tract, pancreas, and other digestive organs; tumors of the oral cavity (*e.g.,* lip, tongue, gum, floor of mouth, palate, parotid gland, salivary glands, tonsil, oropharynx, nasopharynx, puriform sinus, hypopharynx, and other sites of the oral cavity); tumors of the reproductive system (*e.g.,* vulva, vagina, Cervix uteri, uterus, ovary, and other sites associated with female genital organs, placenta, penis, prostate, testis, and other sites associated with male genital organs); tumors of the respiratory tract (*e.g.,* nasal cavity, middle ear, accessory sinuses, larynx, trachea, bronchus and lung, such as small cell lung cancer and non-small cell lung cancer); tumors of the skeletal system (*e.g.,* bone and articular cartilage of limbs, bone articular cartilage and other sites); tumors of the skin (*e.g.,* malignant malonoma of the skin, non-melanoma skin cancer, basal cell carcinoma of skin, squamous cell carcinoma of skin, mesothelioma, Kaposi's sarcoma); and tumors involving other tissues including peripheral nerves and autonomic nervous system, connective and soft tissue, retroperitoneoum and peritoneum, eye and adnexa, thyroid, adrenal gland, and other endocrine glands and related structures, secondary and unspecified malignant neoplasms of lymph nodes, secondary malignant neoplasm of respiratory and digestive systems and secondary malignant neoplasms of other sites.

In embodiments where the toxin moiety is a Shiga toxin B-subunit, or an functional equivalent thereof, compositions and methods of the present invention may be advantageously used for the treatment of cancers that are associated with over expression of the receptor Gb3. Gb3 has been shown to be expressed on a narrow range of committed B cells and associated B-cell lymphomas (J. Gordon et al., Blood, 1983, 62: 910-917; L.J. Murray et al., Int. J. Cancer, 1985, 36: 561-565; M. Mangeney et al., Eur. J. Immunol., 1991, 21: 1131-1140; E; Oosterwijk et al., Int. J; Cancer, 1991, 48: 848-854; A. Kalisiak et al., Int. J. Cancer, 1991, 49: 837-845; E.C. LaCasse et al., Blood, 94: 2901-2910). Ovarian hyperplasias (S. Arab et al., Oncol. Res., 1997, 9: 553-563), cell suspensions obtained from human breast tumors (E.C. LaCasse et al., Blood, 94: 2901-2910), testicular seminomas (C. Ohyama et al., Int. J. Cancer, 1990, 45: 1040-1044), colorectal carcinomas (O. Kovbasnjuk et al., Proc. Natl. Acad. Sci. USA, 2005, 102: 19087-19092), and other small intestine tumors of different origins (E.C. LaCasse et al., Blood, 94: 2901-2910) have been tested positive for Gb3. Gb3 was also observed to be markedly increased in cell lines derived from astrocytomas (S. Arab et al., Oncol. Res., 1999, 11: 33-39). Thus, in certain embodiments, compositions and methods of the present invention are used in the treatment of cancers of the group consisting of lymphomas, ovarian cancers, breast tumors, testicular cancers, colorectal cancers, intestine tumors, and astrocytomas.

Tumors that can be treated using compositions and methods of the present invention may be refractory to treatment with other chemotherapeutics. The term "***refractory***"*,* when used herein in reference to a tumor means that the tumor (and/or metastases thereof), upon treatment with at least one chemotherapeutics other than an inventive composition, shows no or only weak anti-proliferative response (*i.e.,* no or only weak inhibition of tumor growth) after the treatment of such a chemotherapeutic agent - that is, a tumor that cannot be treated at all or only with unsatisfying results with other (preferably standard) chemotherapeutics. The present invention, where treatment of refractory tumors and the like is mentioned, is to be understood to encompass not only (i) tumors where one or more chemotherapeutics have already failed during treatment of a patient, but also (ii) tumors that can be shown to be refractory by other means, *e*.*g*., biopsy and culture in the presence of chemotherapeutics.

In addition, the present invention provides methods for stimulating an immune response in a subject in need thereof, said methods comprising a step of administering to the subject, an effective amount of a composition described herein. The term "***immune response***" includes any immunological response of the subject to an inventive toxin conjugate. In certain preferred embodiments, stimulation of an immune response includes involvement of dendritic cells, *e.g.,* Langerhans cells. Advantageously, the immune response may include, for example, the promotion of T cells, generation of antibodies against the antigen, and/or the presence of the antigen by dendritic cells, *e.g.,* Langerhans cells. The term "stimulating an immune response" also includes initiation or enhancement of an immune response.

### V. Diagnostics

In another aspect, the present invention allows for the non-invasive detection and/or diagnostic of antigen-related states, such as clinical conditions associated with tumors. More specifically, conjugates of the present invention that comprise an imaging moiety can be used to detect, localize, and/or diagnose tumors in *in vitro, in vivo,* and *ex vivo* systems as well as in living patients.

More specifically, the present invention provides methods for detecting the presence of tumors in a system comprising the step of contacting the system with an effective amount of a toxin conjugate described herein, or a composition thereof. The contacting is preferentially carried out under conditions that allow the toxin conjugate to interact with a tumor present in the system so that the interaction results in the binding of the conjugate to the tumor. The toxin conjugate bound to tumor(s) present in the systems is then detected using an imaging technique, and one or more images of at least part of the system are generated. The contacting may be carried out by any suitable method known in the art. For example, the contacting may be carried out by incubation.

The system may be a cell, a biological fluid or a biological tissue, or an animal. When the system is a cell, a biological fluid or a biological tissue, it may originate from a live subject (*e*.*g*., it may be obtained by biopsy). The subject may be a human or another mammal. For example, the cell, biological fluid or biological tissue may originate from a subject suspected of having a clinical condition associated with tumors.

In another aspect of the present invention, the method described above is used for identifying potential therapeutic agents. For example, images of at least part of a cell, biological fluid or biological tissue may be generated before and after contacting the cell, biological fluid or biological tissue with a potential therapeutic agent for the treatment of tumors. Comparison of the "before" and "after" images allows determination of the effects of the agent on tumors present in the system. The invention also includes therapeutic agents identified by this method.

The present invention also provides methods for detecting the presence of tumors (i.e., antigen-related states characterized by the presence of tumors) in a subject. Such methods comprise administering to the subject an effective amount of a toxin conjugate of the invention, or a composition thereof. Administration is preferably carried out under conditions that allow the conjugate (1) to reach the area(s) of the subject's body that may contain tumors and (2) to interact with tumor(s) present so that the interaction results in binding of the toxin conjugate to the tumor(s). After administration of the toxin conjugate and after sufficient time has elapsed for the interaction to take place (for example after between 30 minutes and 48 hours), the conjugate bound to tumor(s) present in the subject is detected using an imaging technique (e.g., MRI, MRS, SPET or PET), and one or more images of at least part of the body of the subject is/are generated.

Such methods may be used to localize tumors in a subject. By comparing the results obtained from a subject suspected to have a tumor and images obtained from studies of clinically healthy individuals, the presence and distribution of tumors can be determined, and the diagnosis confirmed.

Administration of a conjugate, or composition thereof, can be carried out by any suitable method known in the art such as administration by oral and parenteral methods, including intravenous, intraarterial, intrathecal, intradermal and intracavitory administrations, and enteral methods.

Methods of the invention that provide for detecting the presence of tumors in a subject or in a system can be used to follow the progression of clinical conditions associated with tumors. For example, this can be achieved by repeating the method at different time points over of period of time in order to establish a time course for the presence, localization, distribution, and quantification of tumors in a patient.

These methods can also be used to monitor the response of a subject to a treatment for a clinical condition associated with tumors. For example, an image of part of the body of the subject that contains tumors (or an image of part of a cell, biological fluid or biological tissue originating from the subject and containing tumors) is generated before and after submitting the subject to a treatment. Comparison of the "before" and "after" images allows to determine the effects of the treatment on the tumors and therefore to monitor the response of the subject to a particular treatment.

### VI. Pharmaceutical Packs or Kits

In another aspect, the present invention provides a pharmaceutical pack or kit comprising one or more containers (*e.g.,* vials, ampoules, test tubes, flasks or bottles) containing one or more ingredients of an inventive pharmaceutical composition, allowing administration of a conjugate of the present invention.

Different ingredients of a pharmaceutical pack or kit may be supplied in a solid (*e.g.,* lyophilized) or liquid form. Each ingredient will generally be suitable as aliquoted in its respective container or provided in a concentrated form. Pharmaceutical packs or kits may include media for the reconstitution of lyophilized ingredients. Individual containers of the kit will preferably be maintained in close confinement for commercial sale.

In certain embodiments, a pharmaceutical pack or kit includes one or more additional approved therapeutic agent(s) (*e.g.,* one or more anti-cancer agents, as described above). Optionally associated with such container(s) can be a notice or package insert in the form prescribed by a governmental agency regulating the manufacture, use or sale of pharmaceutical or biological products, which notice reflects approval by the agency of manufacture, use or sale for human administration. The notice of package insert may contain instructions for use of a pharmaceutical composition according to methods disclosed herein.

An identifier, e.g., a bar code, radio frequency, ID tags, etc., may be present in or on the kit. The identifier can be used for example, to uniquely identify the kit for purposes of quality control, inventory control, tracking movement between workstations, etc.

### Examples

The following examples describe some of the preferred modes of making and practicing the present invention. However, it should be understood that these examples are for illustrative purposes only and are not meant to limit the scope of the invention. Furthermore, unless the description in an Example is presented in the past tense, the text, like the rest of the specification, is not intended to suggest that experiments were actually performed or data were actually obtained.

### Example 1: Preparation and Testing of an STxB-BSA Conjugate

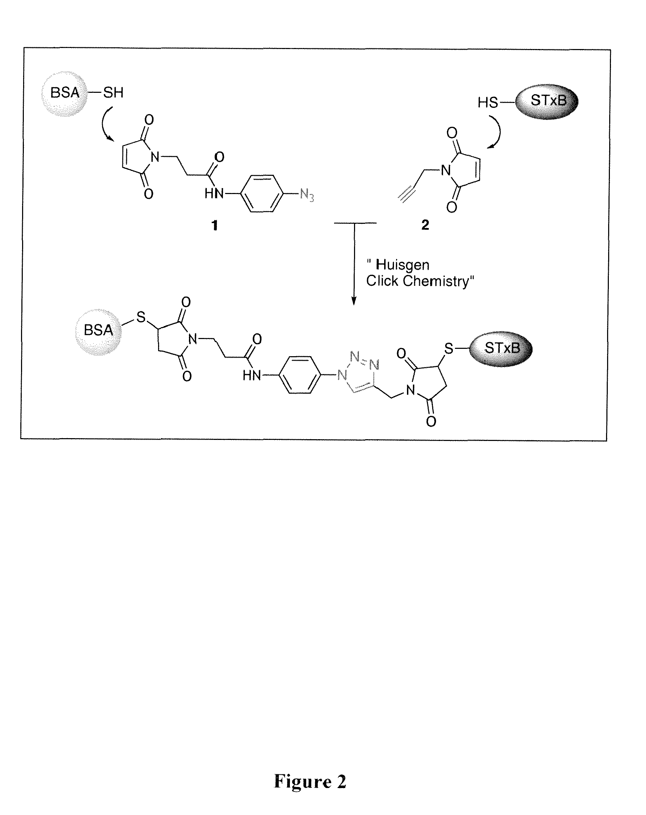

The preparation of a well defined coupling product between STxB and a model protein, bovine serum albumin (BSA) is described in this first example (see Figure 2). BSA was used as the model for several reasons: BSA is a medium size protein which contains several cysteines and that is commercially available. As shown on the scheme presented on Figure 2, preparation of the STxB-BSA conjugate involves labeling of STxB/Cys with a spacer bearing an alkyne, labeling of BSA with a spacer bearing an azido group and reacting the modified STxB/Cys and modified BSA using the Huisgen Click chemistry.

Two functional spacer arm compounds capable of reacting with free sulfhydryl groups on cysteines were synthesized. The first spacer, **1**, is a maleimido-aryl-azide. This compound was used to functionalize BSA (J. Janatova et al., J. Biol. Chem., 1968, 243: 3612-3622; K.L. Heredia et al., J. Am. Chem. Soc., 2005, 127: 16955-16960). The second spacer, **2**, is a maleimido-propargyl. This compound was used to obtain acetylene functionalized STxB. In summary, the maleimido functionality were used to append the respective linkers to cysteine residues of STxB or BSA, while the alkyne and azide functionalities were used to click the two molecules together.

STxB/Cys possesses 5 free cysteines at the C-termini of the B-fragments. The reaction between the free sulfhydryl and the maleimido ring is a spontaneous reaction that does not need any catalyst. One equivalent of the STxB/Cys (0.5 mg/mL) in 20 mM borate buffer pH 9, 10 mM EDTA, and 150 mM NaCl was reacted overnight at room temperature with 3 equivalents of the maleimido-alkyne linker. Linker conjugation was verified by MALDI-TOF analysis.

BSA has several cysteines, which are all in an oxidized form, as verified with Ellman's reagent. To reduce the disulfide bridges, one equivalent of BSA (1 mg/mL) in 50 mM phosphate buffer pH 7.8 150 mM NaCl, was treated with one equivalent of TCEP (tris(2-carboxyethyl)phosphine) at room temperature overnight. The material was then dialyzed against 50 mM phosphate buffer pH 7.8, 150 mM NaCl in order to remove the reducing agent TCEP. One equivalent of reduced BSA (1 mg/mL) was reacted overnight at room temperature with 3 equivalents of the maleimido-azide linker **1**.

STxB-alkyne and BSA-azide were dialyzed extensively against 50 mM phosphate buffer pH 7.8 to remove the excess of linker arms. Equimolar amounts of STxB-alkyne (0.5 mg/mL) and BSA-azide (1 mg/mL) were mixed and then incubated at room temperature in the presence of 4 equivalents of CuSO₄, 16 equivalents of ascorbic acid, and 4 equivalents of bathophenonthrolinesulfonic acid.

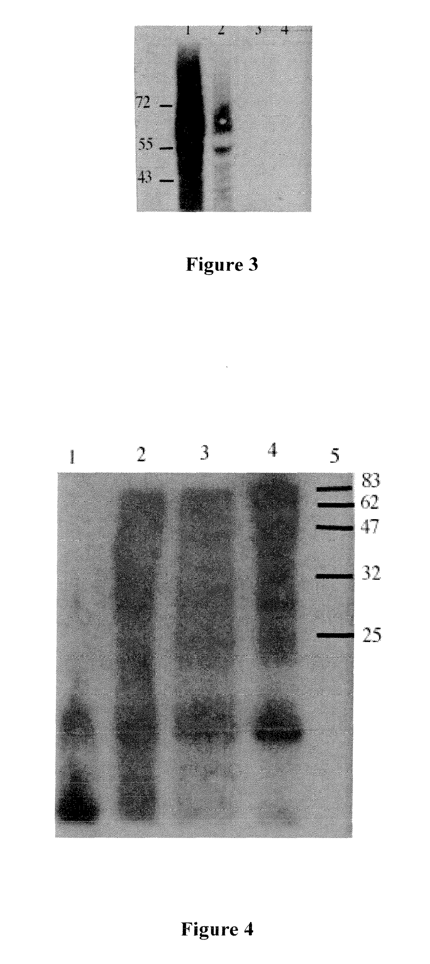

A Western blot analysis using anti-STxB antibody was performed to verify the formation of the coupling agent. A picture of the gel is presented on Figure 3. In the absence of cupper, no coupling was observed (Figure 3, lane 3). A band at molecular weight 70 kDa (corresponding to the molecular weight of the coupling product) was present in the Click reaction containing the catalyst (Figure 3, lanes 1 and 2). As shown by the gel, the reaction worked better at a molecular ratio of 1:1 between STxB-alkyne and BSA-azide (Figure 3, lane 1) than at a molecular ratio of 1:3 (Figure 3, lane 2). Unexpectedly, the Click reaction was observed to work better in a diluted state, as opposed to classical crosslinking schemes. Indeed, as shown in Figure 3, when a Click reaction between STxB-alkyne and BSA-azide in a 1:1 molecular ratio was diluted twofold, the yield of coupling agent was 3-fold higher.

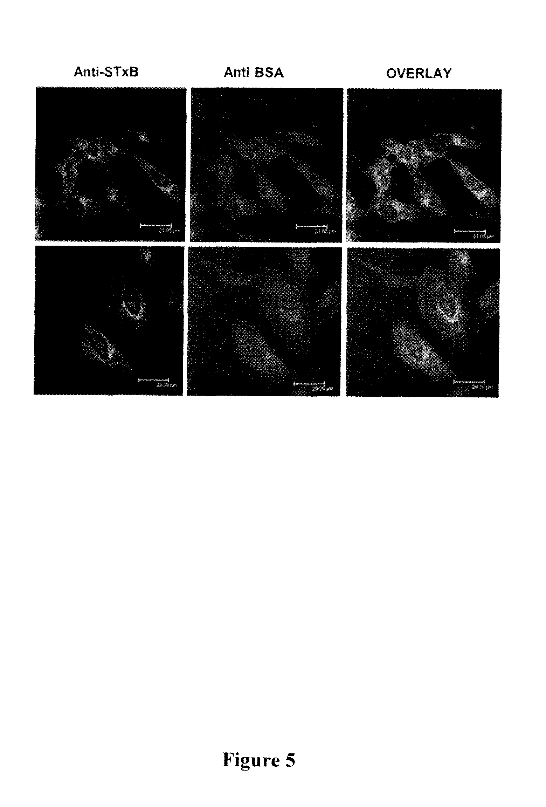

Tests were then carried out to confirm that STxB-BSA, obtained as described above, was functional and could be transported into the Golgi apparatus. HeLa cells were incubated on ice with 200 nM of the coupling agent. After washing, the cells were shifted to 37°C for 45 minutes, fixed, and double labeled for STxB and BSA (see Figure 5). The experiment showed that both labelings perfectly overlapped, thus demonstrating that BSA coupled to STxB *via* Click chemistry could be transported into cells.

### Example 2: Preparation and Testing of an STxB-E7 Conjugate

A second coupling agent was prepared between STxB and the E7 protein. The HPV E7 proteins are small (HPV16 E7 comprising 98 amino acids), zinc binding phosphoproteins which are localised in the nucleus. They are structurally and functionally similar to the E1A protein of subgenus C adenoviruses. The first 16 amino-terminal amino acids of HPV16 E7 contain a region homologous to a segment of the conserved region 1 (CR1) of the E1A protein of subgenus C adenoviruses. The next domain, up to amino acid 37, is homogenous to the entire region 52 (CR2) of E1A. Genetic studies have established that these domains are required for cell transformation *in vitro,* suggesting similarities in the mechanism of transformation by these viruses. The CR2 homology region contains the LXCXE motif (residues 22-26) involved in binding to the tumor suppressor protein pRb. This sequence is also present in SV40 and polyoma large T antigens. The high risk HPV E7 proteins (of, for example, types 16 and 18) have an approximately ten-fold higher affinity for pRb protein than the low risk HPV E7 proteins (of, for example, type 6). Association of the E7 protein with pRb promotes cell proliferation by the same mechanism as the E1A proteins of adenoviruses and SV40 large T antigen. Recent studies have shown that E7 promotes degradation of Rb family proteins rather than simply inhibition of their function by complex formation. The CR2 region also contains the casein kinase II phosphorylation site (residues 31 and 32). The remaining 61 amino acids of E7 protein have very little similarity to E1A, however a sequence CXXC involved in zinc binding is present in both proteins. The E7 protein contains two of these motifs which mediate dimerization of the protein. Mutation in one of the two Zn binding motifs destroys transforming activity, although this mutant is able to associate with Rb protein. Therefore, dimerization may be important for the transforming activity of E7.

***Functionalization of E7*:** E7 protein at a concentration of 0.5 mg/mL was dialyzed against 20 mM Pi buffer pH 7.5. The dialyzed protein was then functionalized with the NHSPEGN3 linker (N-hydroxy succinimide (NHS) polyethylene glycol (PEG) azide (N3) linker). Two different E7:linker ratios were used: 1:9 and 1:40. The coupling reaction was carried out overnight at room temperature, and the reaction mixture was then extensively dialyzed to remove the linker against 20 mM Pi buffer pH 7.8.

***Click Reaction E7-STxB*:** One equivalent of STxB-alkyne (4.5 mg/mL), prepared as described above, was reacted with one equivalent of E7-azide (0.5 mg/mL) in presence of an excess of ascorbic acid and cupper, with bathophenontrolin as a cupper ligand. The reaction was carried out overnight at room temperature and a sample was used to perform a Western blot using antibodies against STxB and E7 proteins.

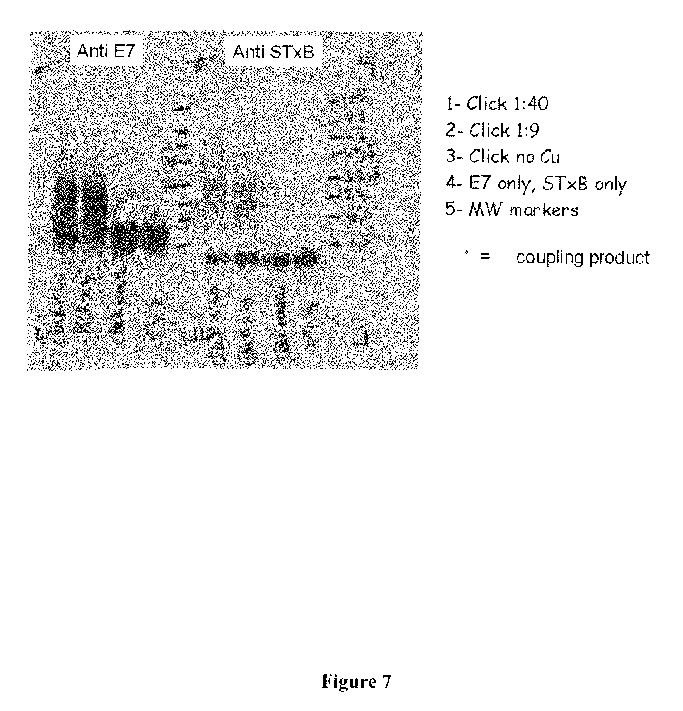

***Quantitation of the STxB-E7** Coupling:* The efficiency of coupling of the Click reaction was then assessed. Two Click reactions *(i.e.,* one using E7 functionalized with 3 equivalents of linker, the other using E7 functionalized with 9 equivalents of linker) and one Click reaction in the absence of catalyst were run on a gel, and then blotted on a nitrocellulose membrane (see Figure 7). The presence of two bands recognized by both anti-StxB and anti-E7 polyclonal antibodies demonstrate the formation of STxB-E7. The two bands show up with apparent molecular weights of 23 and 30 kDa, demonstrating the presence of an adduct of molecular weight of 23 kDa comprising one molecule of E7 and one molecular of STxB and an adduct of molecular weight of 30 kDa comprising two molecules of E7 and one molecule of StxB. The two bands were not observed when the Click reaction was performed in the absence of cupper, which also confirms the nature of the bands.

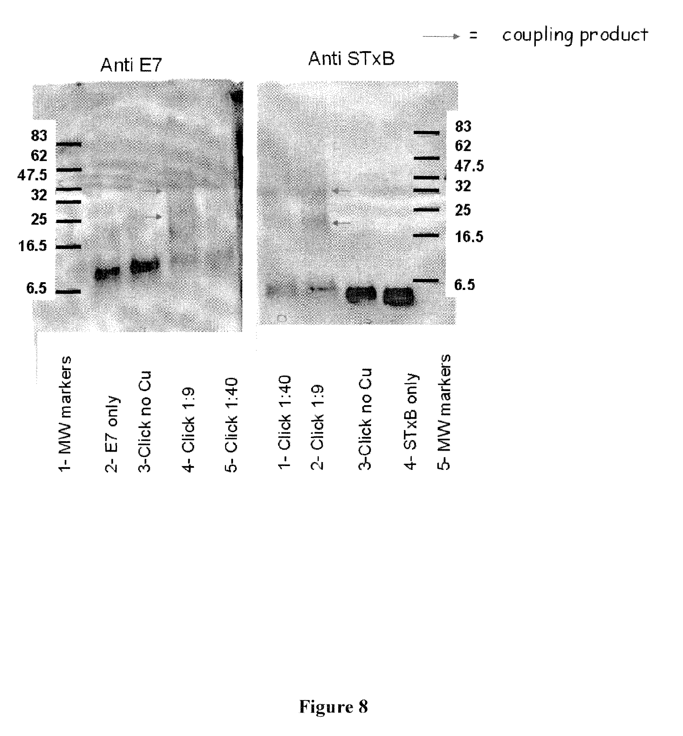

Western blot was also used to quantitate the formation of coupling products between STtxB and E7 using the ECF method (enhanced chemifluorescence). Again, two Click reactions *(i.e.,* one using E7 functionalized with 3 equivalents of linker, the other using E7 functionalized with 9 equivalents of linker) and one Click reaction in the absence of catalyst were run on a gel, and the intensity of the bands was quantitated (see Figure 8).

The calculations show that the amount of free monomeric STxB (uncoupled STxB) was around 56% for the Click reaction with the 1:9 ratio and 43% for the Click reaction with the 1:40 ratio, while almost 2/3 of E7 was coupled to STxB in the Click reaction with the 1:9 ratio. It can be considered that coupling was quantitative. Indeed, STxB is a pentamer of 5 B-fragments. A coupling of 43 to 56% of the monomers translates into the statistical presence of 2-3 E7 proteins per STxB pentamer. Similarly, E7 exists in solution as soluble inclusion bodies (Y. Nominé et al., Protein Eng., 2001, 14: 297-305). With a coupling efficiency of more than 50%, each of these carries at least one STxB modification.

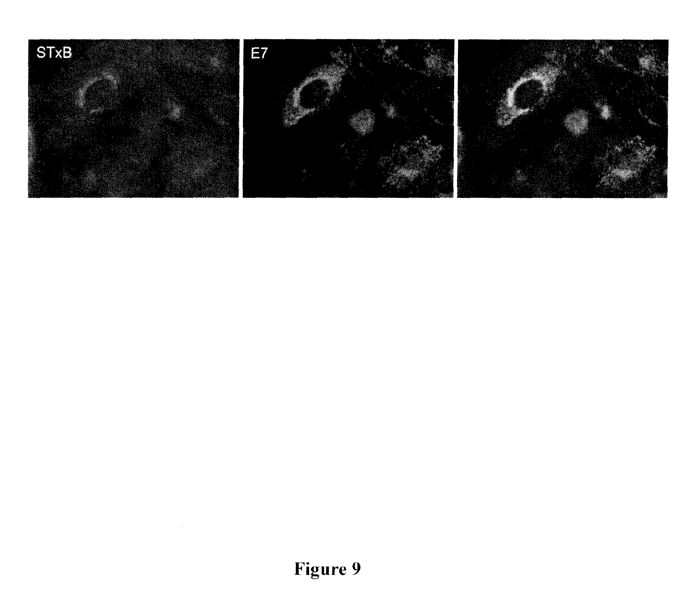

***In vitro Testing of STxB-E7*:** STxB-E7 was then tested for retrograde transport *in vitro.* Products from Click reactions performed in 3 different conditions were used, *i.e.,* Click reaction with E7:linker ratio of 1:9, Click reaction with E7:linker ratio of 1:40, and Click reaction in the absence of cupper. Anti-STxB and anti-E7 antibodies were used for IF (immunofluorescence). A co-localization of the STxB and E7 proteins was observed in the Golgi network for the products obtained in the Click 1:9 and 1:40, while the products obtained in the absence of cupper did not show any evidence of internalization of the E7 protein (see Figure 9). The coupling products (1 µM) were incubated for 30 minutes with HeLa cells on ice. After washing, the cells were shifted for 45 minutes to 37°C, fixed, and stained with the indicated antibodies.

### Example 3: Preparation and Testing of Conjugate STxB-Her2/neu

The cDNA coding for the extracellular domain of Her2/neu (see sequence below - SEQ ID 1) is modified at the 5' end to encode an additional Cys. The protein is expressed in an appropriate host to assure formation of disulfide bonds and glycosylation. Appropriate hosts are Pichia pasteuris and mammalian cell lines such as Chinese hamster ovary cells. The purified Her2/neu is reacted at 0.5 mg/mL with maleimido-NHS-N3 linker at a molar ratio of 1:3. After removal of the non reacted liker, one equivalent of Her2/neu-N3 protein (0.5 mg/mL) is then reacted with one equivalent of STxB-alzine (4.5 mg/mL) in presence of an excess of ascorbic acid and cupper, with bathophenontrolin as a cupper ligand. The reaction is carried out overnight at room temperature and a sample is used to perform a Western blot using antibodies against STxB and Her2/neu proteins.

### SEQ ID 1:

CTQVCTGTD MKLRLPASPE THLDMLRHLY QGCQVVQGNL ELTYLPTNAS LSFLQDIQEV QGYVLIAHNQ VRQVPLQRLR IVRGTQLFED NYALAVLDNG DPLNNTTPVT GASPGGLREL QLRSLTEILK GGVLIQRNPQ LCYQDTILWK DIFHKNNQLA LTLIDTNRSR ACHPCSPMCK GSRCWGESSE DCQSLTRTVC AGGCARCKGP LPTDCCHEQC AAGCTGPKHS DCLACLHFNH SGICELHCPA LVTYNTDTFE SMPNPEGRYT FGASCVTACP YNYLSTDVGS CTLVCPLHNQ EVTAEDGTQR CEKCSKPCAR VCYGLGMEHL REVRAVTSAN IQEFAGCKKI FGSLAFLPES FDGDPASNTA PLQPEQLQVF ETLEEITGYL YISAWPDSLP DLSVFQNLQV IRGRILHNGA YSLTLQGLGI SWLGLRSLRE LGSGLALIHH NTHLCFVHTV PWDQLFRNPH QALLHTANRP EDECVGEGLA CHQLCARGHC WGPGPTQCVN CSQFLRGQEC VEECRVLQGL PREYVNARHC LPCHPECQPQ NGSVTCFGPE ADQCVACAHY KDPPFCVARC PSGVKPDLSY MPIWKFPDEE GACQPCPINC THSCVDLDDK GCPAEQRASP LHHHHHH

### Other Embodiments

Other embodiments of the invention will be apparent to those skilled in the art from a consideration of the specification or practice of the invention disclosed herein. It is intended that the specification and examples be considered as exemplary only, with the true scope of the invention being indicated by the following claims.

## Claims

1. Toxin conjugate comprising at least one toxin moiety covalently bound to at least one bioactive moiety, wherein covalent binding between the toxin moiety and bioactive moiety results from a [3+2] cycloaddition.

2. The toxin conjugate according to claim 1, wherein the toxin moiety is Shiga toxin B-subunit, or a functional equivalent thereof, and the bioactive moiety comprises the extracellular domain of antigen Her2/neu.

3. The toxin conjugate according to claim 1 or claim 2, wherein the [3+2] cycloaddition occurs between a first reactive unsaturated group on the toxin moiety and a second reactive unsaturated group on the bioactive moiety.

4. The toxin conjugate according to claim 3, wherein the first reactive unsaturated group comprises a 1,3-dipole and the second reactive unsaturated group comprises a dipolarophile or wherein the first reactive unsaturated group comprises a dipolarophile and the second reactive unsaturated group comprises a 1,3-dipole.

5. The toxin conjugate according to claim 4, wherein the 1,3-dipole comprises a nitrile oxide, an azide, a nitrone or a nitrile imine.

6. The toxin conjugate according to claim 4, wherein the dipolarophile comprises an alkene or an alkyne.

7. The toxin conjugate according to claim 4, wherein the 1,3-dipole comprises an azido group and the dipolarophile comprises an ethynyl group.

8. The toxin conjugate according to anyone of claims 1-7, wherein the [3+2] cycloaddition occurs in the presence of Cu(I).

9. The toxin conjugate according to claim 1, wherein the toxin moiety is selected from the group consisting of anthrax toxin, pertussin toxin, diphtheria toxin, chlorotoxin, botulinum toxin, cholera toxin, *Escherichia coli* heat-labile enterotoxin, pH-sensitive toxins, and functional equivalents thereof.

10. The toxin conjugate according to claim 1, wherein the toxin moiety is selected from the group consisting of Shiga toxin B-subunit, verotoxin B-subunit, and any functional equivalents thereof.

11. The toxin conjugate according to anyone of claims 1, and 3-10, wherein the bioactive moiety comprises a therapeutic moiety selected from the group consisting of antigens, antigen epitopes, therapeutic antibodies, chemotherapeutics, nucleic acids, cytokines, growth factors, hormones, small molecules, and any combinations thereof.

12. The toxin conjugate according to claim 11, wherein the therapeutic moiety is an antigen or an antigen epitope.

13. The toxin conjugate according to claim 12, wherein the therapeutic moiety comprises a tumor antigen, a viral antigen, a bacterial antigen, a tumor antigen epitope, a viral antigen epitope, or a bacterial antigen epitope.

14. The toxin conjugate according to claim 13, wherein the antigen is selected from the group consisting of E6, E7, antigens from the Mage family, Her2/neu, EGFRVIII, survivin, telomerase, WT1, ESAT6, Hepatitis B Virus (HBV) antigens L1 and L2, and any active fragments thereof.

15. The toxin conjugate according to anyone of claims 1 and 3-10, wherein the bioactive moiety comprises an imaging moiety selected from the group consisting of entities detectable by MRI, entities detectable by MRS, entities detectable by SPECT; and entities detectable by PET.

16. Composition comprising a toxin conjugate according to anyone of claims 1-15.

17. The composition of claim 16 further comprising a pharmaceutically acceptable carrier.

18. Medicament comprising an effective amount of a composition according to claim 16 or claim 17.

19. Use of a composition according to claim 16 or claim 17 for the manufacture of a medicament for the treatment of an antigen-related state in a subject, said antigen-related state being a tumor or an infection.

20. The use according to claim 19, wherein the composition is to be administered to said subject in an effective amount for stimulating an immune response against the antigen in said subject, thereby treating said antigen-related state in said subject.

21. The use according to claim 20, wherein stimulating an immune response comprises stimulating dendritic cells.

22. The use according to claim 21, wherein stimulating an immune response comprises eliciting an antigen specific CD 8 response.

23. Method of preparing a toxin conjugate, the method comprising steps of:
providing a toxin moiety comprising a first reactive unsaturated group; and
contacting the toxin moiety with a bioactive moiety comprising a second reactive unsaturated group, such that a [3+2] cycloaddition occurs between the first and second unsaturated groups.

24. The method according to claim 23, wherein the first reactive unsaturated group comprises a 1,3-dipole and the second reactive unsaturated group comprises a dipolarophile or wherein the first reactive unsaturated group comprises a dipolarophile and the second reactive unsaturated group comprises a 1,3-dipole.

25. The method according to claim 24, wherein the 1,3-dipole comprises a nitrile oxide, an azide, a nitrone, or a nitrile imine.

26. The method according to claim 24, wherein the dipolarophile comprises an alkene or an alkyne.

27. The method according to claim 24, wherein the 1,3-dipole comprises an azido group and the dipolarophile comprises an ethynyl group.

28. The method according to anyone of claims 23-27, wherein the step of contacting is performed in the presence of Cu(I).

29. The method according to anyone of claims 23-27, wherein the toxin moiety is selected from the group consisting of anthrax toxin, pertussin toxin, diphtheria toxin, chlorotoxin, botulinum toxin, cholera toxin, *Escherichia coli* heat-labile enterotoxin, pH-sensitive toxins, and functional equivalents thereof.

30. The method according to anyone of claims 23-27, wherein the toxin moiety is selected from the group consisting of Shiga toxin B-subunit, verotoxin B-subunit, and any functional equivalents thereof.

31. The method according to anyone of claims 23-27, wherein the bioactive moiety comprises a therapeutic moiety selected from the group consisting of antigens, antigen epitopes, therapeutic antibodies, chemotherapeutics, nucleic acids, cytokines, growth factors, hormones, small molecules, and any combinations thereof.

32. The method according to claim 31, wherein the therapeutic moiety comprises an antigen or antigen epitope.

33. The method according to claim 32, wherein the therapeutic moiety comprises a tumor antigen, a viral antigen, a bacterial antigen, a tumor antigen epitope, a viral antigen epitope or a bacterial antigen epitope.

34. The method according to claim 33, wherein the antigen is selected from the group consisting of E6, E7, antigens from the Mage family, Her2/neu, EGFRVIII, survivin, telomerase, WT1, ESAT6, Hepatitis B Virus (HBV) antigens L1 and L2, and any active fragments thereof.

35. The method according to anyone of claims 23-27, wherein the bioactive moiety comprises an imaging moiety selected from the group consisting of entities detectable by MRI, entities detectable by MRS, entities detectable by SPECT; and entities detectable by PET.
